# EUROPÄISCHE PATENTSCHRIFT

(11) **EP 2 181 696 B1**
(45) Veröffentlichungstag und Bekanntmachung des Hinweises auf die Patenterteilung: **12.04.2017**
(21) Anmeldenummer: 09173142.2
(22) Anmeldetag: 15.10.2009
(51) Int. Cl.: A61K 8/368, A61K 8/73, A61K 8/81, A61Q 19/00, A61P 17/10

(54) **Anti Pickel Hautbehandlungsmittel**
Anti-spot skin treatment
Moyen de traitement de la peau contre les boutons

(30) Priorität: 30.10.2008 DE 102008053884
(43) Veröffentlichungstag der Anmeldung: 05.05.2010
(73) Patentinhaber: Henkel AG & Co. KGaA, 40589 Düsseldorf (DE)
(72) Erfinder: Struwe, Alexandra, 47877 Willich (DE); Waldmann-Laue, Marianne, 40789 Monheim (DE); Bähren, Annika, 41363 Jüchen (DE)

(56) Entgegenhaltungen:
- EP-A1- 0 281 812
- EP-A1- 2 182 027
- WO-A2-2007/108883
- CN-A- 1 528 343
- DE-A1-102005 016 687
- US-A1- 2006 204 530
- US-B1- 7 189 406

## Beschreibung

Die Erfindung betrifft Zubereitungen zur Hautpflege, die aufgrund von speziellen Inhaltsstoffen eine ausgezeichnete antibakterielle Wirkung besitzen.

Es hat nicht an Versuchen gefehlt, Akne zu bekämpfen, und eine Vielzahl von Agentien wird zu diesem Zweck in Zubereitungen zur Reinigung und Bekämpfung der Bakterien, die für Akne und unreine Haut verantwortlich sind, eingesetzt. Als antibakterielle Mittel finden beispielsweise Tri-closan, Benzoylperoxid oder Chlorhexidin Verwendung. Der Einsatz dieser Produkte kann jedoch in Einzelfällen mit Nachteilen verbunden sein. Chlorierte organische Verbindungen, wie Triclosan oder Chlorhexidin, sind aus ökologischen und gesetzlichen Gründen zu vermeiden. Benzoylperoxid ist ein reaktiver, allerdings bakterien-unspezifischer Wirkstoff, der zu Hautirritationen führen kann. Es besteht daher nach wie vor das Bedürfnis, Akne und unreine Haut zu bekämpfen und Wirkstoffe oder Wirkstoffkombinationen dafür bereitzustellen, die frei von den genannten Nachteilen sind. In den vergangenen Jahren hat sich bei vielen Kosmetikanwendern, insbesondere bei Frauen in der Zeit nach der Pubertät bis zum Beginn der Wechseljahre, das Phänomen bemerkbar, dass die Probleme mit unreiner und/oder fettiger Haut und der Neigung zu Akne und Pickelbildung über die Pubertät hinaus bestehen, gleichzeitig aber das Erscheinungsbild der Hautalterung immer stärker hervortritt.

Pflegeprodukte, die das Erscheinungsbild der Hautalterung, wie insbesondere Fältchen, feine Linien und (erste) Falten, vor allem aber auch den zunehmenden Verlust an Hautfeuchtigkeit, behandeln und mildern sollen, enthalten häufig einen höheren Anteil an pflegenden Fetten und Ölen, die den Feuchtigkeitsgehalt der Haut steigern und die Elastizität der Haut verbessern sollen. Diese Öle und Fette allerdings sind häufig komedogen und verschlechtern das Hautbild der Hautpartien, die von Pickeln und Mitessern betroffen sind, welche meist in der sogenannten T-Zone (Stirn, Nase, Kinn) angesiedelt sind. Die Stirn ist allerdings auch besonders von der Bildung von Fältchen und Linien betroffen, so dass deren Behandlung mit einem feuchtigkeitssteigernden Produkt eigentlich zwingend erforderlich wäre.

Die Behandlung von Hautunreinheiten und fettiger Haut kann auch mit anderen Mitteln als den oben genannten Wirkstoffen Triclosan, Benzoylperoxid oder Chlorhexidin erfolgen, z. B. mit ethanolischen oder glycolischen Lösungen. Diese führen aber insbesondere bei der nachpubertären Haut häufig zu einer zu starken Entfettung, unangenehmem, spannenden Hautgefühl und zu Reizungen.

Die Aufgabe der vorliegenden Erfindung bestand darin, Zubereitungen zur antibakteriellen Hautpflege und -reinigung bereitzustellen, die Akne und unreine Haut verursachende Bakterien wirksam und spezifisch bekämpfen und gegen Akne und unreine Haut wirksam sind. Dabei sollten die Nachteile des Einsatzes bekannter Wirkstoffe, wie Triclosan, Benzoylperoxid oder Chlorhexidin, vermieden werden können. Insbesondere sollten Hautunverträglichkeiten und -reizungen sowie die Schädigung der hautpositiven Keime in der Hautflora vermieden werden.

Es wurde nun gefunden, dass sich eine Kombination aus niedrigdosierten an sich bekannten Wirkstoffen synergistisch verhält, so dass mit insgesamt deutlich verringerter Einsatzmenge hoch wirksame Mittel bereitgestellt werden können, die sehr gut gegen Pickel wirken und dennoch äußerst gut verträglich sind.

Gegenstand der vorliegenden Erfindung ist in einer ersten Ausführungsform eine kosmetische oder dermatologische topische Zusammensetzung mit einem Gehalt an Benzoesäure und/oder ihren Salzen und Salicylsäure und/oder ihren Salzen, dadurch gekennzeichnet, dass
a) die Gesamtmenge an Benzoesäure und Benzoaten in der Zusammensetzunge bezogen auf das Gewicht der Zusammensetzung 0,2 bis 0,7 Gew.-% beträgt und
b) die Gesamtmenge an Salicylsäure und Salicylaten in der Zusammensetzunge bezogen auf das Gewicht der Zusammensetzung 0,2 bis 0,7 Gew.-% beträgt und
c) das Gewichtsverhältnis der Gesamtmenge an Benzoesäure und Benzoaten zur Gesamtmenge an Salicylsäure und Salicylaten im Bereich von 3 : 2 bis 2 : 3 liegt,
wobei sie bezogen auf ihr Gewicht 0,3 bis 0,65 Gew.-% Natriumbenzoat enthält und wobei sie bezogen auf ihr Gewicht 0,3 bis 0,65 Gew.-% Natriumsalicylat enthält. Die erfindungsgemäßen Zusammensetzungen können in verschiedenen Angebotsformen konfektioniert werden, beispielsweise als (ggf. öl- und fettfreies) Gel, als Creme, in Stiftform, als flüssige oder gelförmige Roll on-Applikation, als getränktes flexibles Substrat (Anti-Pickel-Pad), aber auch als Puder oder Spray.

Erfindungsgemäße Mittel können in fester, halbfester, flüssiger, disperser, emulgierter, suspendierter, oder gelförmiger Form vorliegen. Weiterhin können die erfindungsgemäßen Mittel als Aerosol konfektioniert sein, das heißt, sie sind in einem Druckbehälter verpackt, aus dem sie mit Hilfe eines Treibmittels versprüht werden können. Weiterhin können die erfindungsgemäßen Mittel als treibgasfreies Pumpspray versprüht werden.

Ganz besonders bevorzugt werden erfindungsgemäße Mittel als verdickte wässrige Zusammensetzungen bereitgestellt. Ein die Wirksamkeit der erfindungsgemäßen Kombination besonders unterstützender kosmetischer Träger wird weiter unten offenbart.

Erfindungsgemässe Zusammensetzungen sind dadurch gekennzeichnet, dass sie bezogen auf ihr Gewicht 0,3 bis 0,65 Gew.-%, vorzugsweise 0,35 bis 0,6 Gew.-%, besonders bevorzugt 0,4 bis 0,6 Gew.-% und insbesondere 0,45 bis 0,5 Gew.-% Natriumbenzoat enthalten.

Erfindungsgemässe Zusammensetzungen sind dadurch gekennzeichnet, dass sie bezogen auf ihr Gewicht 0,3 bis 0,65 Gew.-%, bevorzugt 0,35 bis 0,6 Gew.-%, besonders bevorzugt 0,4 bis 0,6 Gew.-% und insbesondere 0,45 bis 0,5 Gew.-% Natriumsalicylat enthalten.

Erfindungsgemässe Zusammensetzungen enthalten sowohl Natriumbenzoat als auch Natriumsalicylat. In noch weiter bevorzugten erfindungsgemäßen Zusammensetzungen ist zusätzlich zu den beiden genannten Stoffen keine weitere Substanz aus der Gruppe der Benzoate und Salicylate enthalten.

Es ist erfindungsgemäß Benzoesäure/Benzoate und Salicylsäure/Salicylate innerhalb enger Verhältnisse zueinander einzusetzen. Hier sind erfindungsgemäße Zusammensetzungen bevorzugt, bei denen das Gewichtsverhältnis der Gesamtmenge an Benzoesäure und Benzoaten zur Gesamtmenge an Salicylsäure und Salicylaten im Bereich von 3 : 2 bis 2 : 3, vorzugsweise von 5 : 4 bis 4 : 5 und insbesondere von 1,1 : 1 bis 1: 1,1 beträgt.

Wie bereits erwähnt, sind bevorzugte erfindungsgemäße Zusammensetzungen wässrige, verdickte Mittel. Ganz besonders bevorzugte erfindungsgemäße Zusammensetzungen enthalten bezogen auf ihr Gewicht
a) 20 bis 95 Gew.-% Wasser und
b) Dehydroxanthan Gum und
c) mindestens ein vernetztes Copolymer aus Vinylpyrrolidon und 2-Methyl-2[(1-oxo-2-propenyl)amino]-1-propansulfonsäure, das teilweise oder vollständig neutralisiert ist,
d) mindestens einen polymeren Verdicker in Form eines inversen, auto-inversiblen Polymerlatex, enthaltend eine Ölphase, eine Wasserphase, mindestens einen Öl-in-Wasser-Emulgator und mindestens einen verzweigten oder vernetzten Polyelektrolyten, wobei der Polyelektrolyt ausgewählt ist aus
   i) einem Homopolymer, dessen Monomer eine schwache Säurefunktion enthält, die teilweise oder vollständig neutralisiert ist,
   ii) einem Copolymer, das mindestens ein Monomer mit einer starken Säurefunktion sowie ein neutrales Monomer oder ein Monomer mit einer schwachen Säurefunktion enthält.

Der spezielle Träger dieser bevorzugten Ausführungsform führt zu einer angenehmen Applikation der erfindungsgemäßen Zusammensetzungen ohne Verlaufen, Tropfen oder Kleben. Zudem wird die Wirksamkeit der erfindungsgemäßen Zusammensetzung durch diesen Träger weiter erhöht.

Das Verdickersystem dieser bevorzugten Ausführungsform eignet sich hervorragend zur Verdickung wässrig-basierter Systeme, so dass die erfindungsgemäßen Zusammensetzungen dieser bevorzugten Ausführungsform - bezogen auf ihr Gewicht - 20 bis 95 Gew.-% Wasser enthalten. Bevorzugte erfindungsgemäße Zusammensetzungen sind dadurch gekennzeichnet, dass sie bezogen auf ihr Gewicht 30 bis 94 Gew.-%, vorzugsweise 35 bis 92,5 Gew.-%, besonders bevorzugt 40 bis 90 Gew.-%, weiter bevorzugt 50 bis 87,5 Gew.-% und insbesondere 60 bis 85 Gew.-% Wasser enthalten.

Als ersten Bestandteil der Verdickerkombination enthalten die erfindungsgemäßen Zusammensetzungen dieser bevorzugten Ausführungsform Dehydroxanthan Gum (dehydratisiertes Xanthan-Gum mit der INCI-Bezeichnung Dehydroxanthan Gum). Die Herstellung von dehydratisiertem Xanthan-Gum ist beispielsweise aus der Offenlegungsschrift JP 07278202 A bekannt. Dehydratisiertes Xanthan-Gum mit der INCI-Bezeichnung Dehydroxanthan Gum ist unter dem Handelsnamen Amaze^{®} XT von National Starch erhältlich. Von National Starch sind auch zahlreiche Formulierungsbeispiele mit Amaze^{®} XT veröffentlicht, denen der Fachmann jedoch keinen Hinweis auf eine besondere Eignung und besondere Vorteile des Rohstoffs Amaze^{®} XT zur Verdickung saurer Zusammensetzungen entnehmen kann.

Erfindungsgemäß besonders bevorzugte Zusammensetzungen dieser bevorzugten Ausführungsform sind dadurch gekennzeichnet, dass sie bezogen auf ihr Gewicht 0,05 bis 5 Gew.-%, vorzugsweise 0,1 bis 4 Gew.-%, besonders bevorzugt 0,25 bis 3 Gew.-%, weiter bevorzugt 0,5 bis 2,5 Gew.-% und insbesondere 1 bis 2 Gew.-% Dehydroxanthan Gum b) enthalten.

Das in den erfindungsgemäßen Zusammensetzungen dieser bevorzugten Ausführungsform als zweiter Bestandteil der Verdickerkombination enthaltene vernetzte Copolymer aus Vinylpyrrolidon und 2-Methyl-2[(1-oxo-2-propenyl)-amino]-1-propansulfonsäure, das teilweise oder vollständig neutralisiert ist, ist erhältlich durch Copolymerisation dieser beiden Monomere in Gegenwart von Ammoniumhydroxid und einem Vernetzungsmittel. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Bevorzugte erfindungsgemäße Zusammensetzungen dieser bevorzugten Ausführungsform enthalten bezogen auf ihr Gewicht 0,05 bis 3 Gew.-%, vorzugsweise 0,1 bis 2 Gew.-%, besonders bevorzugt 0,2 bis 1,5 Gew.-%, weiter bevorzugt 0,25 bis 1,25 Gew.-% und insbesondere 0,5 bis 1 Gew.-% mindestens eines vernetzten Copolymers c) aus Vinylpyrrolidon und 2-Methyl-2[(1-oxo-2-propenyl)amino]-1-propansulfonsäure, das teilweise oder vollständig neutralisiert ist.

Als dritten Bestandteil der Verdickerkombination enthalten die erfindungsgemäßen Zusammensetzungen dieser bevorzugten Ausführungsform mindestens einen polymeren Verdicker d) in Form eines inversen, auto-inversiblen Polymerlatex, enthaltend eine Ölphase, eine Wasserphase, mindestens einen ÖI-in-Wasser-Emulgator und mindestens einen verzweigten oder vernetzten Polyelektrolyten, der ein Copolymer darstellt, das mindestens ein Monomer mit einer starken Säurefunktion sowie mindestens ein weiteres Monomer aufweist, das neutral ist oder eine schwache Säurefunktion enthält.

Besonders bevorzugte erfindungsgemäße Zusammensetzungen sind dadurch gekennzeichnet, dass sie bezogen auf ihr Gewicht 0,1 bis 5 Gew.-%, vorzugsweise 0,25 bis 4 Gew.-%, besonders bevorzugt 0,5 bis 3 Gew.-%, weiter bevorzugt 0,75 bis 2,5 Gew.-% und insbesondere 1 bis 2 Gew.-% mindestens eines polymeren Verdickers d) in Form eines inversen, auto-inversiblen Polymerlatex enthalten.

Bevorzugte Polyelektrolyten sind aus zwei, drei, vier, fünf oder sechs verschiedenen Monomeren aufgebaut, von denen mindestens ein Monomer eine starke Säurefunktion aufweist und das mindestens eine weitere Monomer neutral ist oder eine schwache Säurefunktion aufweist.
Bevorzugt sind die Monomerkombinationen
starke Säurefunktion/neutral,
starke Säurefunktion/schwache Säurefunktion,
starke Säurefunktion/schwache Säurefunktion/neutral
starke Säurefunktion/schwache Säurefunktion 1/schwache Säurefunktion 2/neutral,
starke Säurefunktion/schwache Säurefunktion/neutral 1/neutral 2,
starke Säurefunktion/schwache Säurefunktion 1/schwache Säurefunktion 2/neutral 1/neutral 2.

Die oben angegebene Reihenfolge der Auflistung der Monomerbausteine gibt dabei nicht notwendigerweise die tatsächliche Reihenfolge der Monomerbausteine im Polyelektrolytmolekül wieder.

In einer bevorzugten Zusammensetzung dieser bevorzugten Ausführungsform ist die schwache Säurefunktion des Polyelektrolytmonomeren im polymeren Verdicker d) eine Carboxylgruppe -COOH, die teilweise oder vollständig neutralisiert ist. Aus der partiellen Neutralisation der schwachen Säurefunktion resultieren der neutralisierte Monomerbaustein und der nicht-neutralisierte, saure Monomerbaustein, beispielsweise Acrylsäure und Natriumacrylat. Beide Monomere werden im Sinne der vorliegenden Anmeldung als gleich angesehen. Dementsprechend werden beide Monomere im Sinne der vorliegenden Anmeldung als "Monomer mit schwacher Säurefunktion" angesehen.

Besonders bevorzugte Beispiele für solche schwach sauren Polyelektrolytmonomeren sind Acrylsäure, Methacrylsäure, Itaconsäure und Maleinsäure. Bevorzugte erfindungsgemäße Zusammensetzungen sind daher dadurch gekennzeichnet, dass die schwache Säurefunktion des Polyelektrolytmonomeren eine Carboxylgruppe -COOH darstellt, die teilweise oder vollständig neutralisiert ist, wobei weiter bevorzugte erfindungsgemäße Zusammensetzungen dieser bevorzugten Ausführungsform dadurch gekennzeichnet sind, dass die Monomere des Polyelektrolyten, die mit der schwach sauren -COOH-Gruppe funktionalisiert sind, ausgewählt sind aus Acrylsäure, Methacrylsäure, Itaconsäure und Maleinsäure, die teilweise oder vollständig neutralisiert sind. In einer weiteren bevorzugten Zusammensetzungen dieser bevorzugten Ausführungsform ist die starke Säurefunktion des Polyelektrolytmonomeren im polymeren Verdicker d) ausgewählt aus einer Sulfonsäuregruppe -SO₃H und einer Phosphonsäuregruppe, die teilweise oder vollständig neutralisiert sind.

Ein besonders bevorzugtes Polyelektrolytmonomer mit starker Säurefunktion ist 2-Methyl-2[(1-oxo-2-propenyl)amino]-1-propansulfonsäure, die teilweise oder vollständig neutralisiert ist, so dass bevorzugte erfindungsgemäße Zusammensetzungen dieser bevorzugten Ausführungsform dadurch gekennzeichnet sind, dass das Polyelektrolytmonomer mit starker Säurefunktion im polymeren Verdicker d) ausgewählt ist aus 2-Methyl-2[(1-oxo-2-propenyl)amino]-1-propansulfonsäure, die teilweise oder vollständig neutralisiert ist.

Aus der partiellen Neutralisation der starken Säurefunktion resultieren der neutralisierte Monomerbaustein und der nicht-neutralisierte, saure Monomerbaustein, beispielsweise 2-Methyl-2[(1-oxo-2-propenyl)amino]-1-propansulfonsäure und Natrium-2-methyl-2[(1-oxo-2-propenyl)amino]-1-propansulfonat. Beide Monomere werden im Sinne der vorliegenden Anmeldung als gleich angesehen. Dementsprechend werden beide Monomere im Sinne der vorliegenden Anmeldung als "Monomer mit starker Säurefunktion" angesehen.

Bevorzugte erfindungsgemäße Zusammensetzungen dieser bevorzugten Ausführungsform sind daher dadurch gekennzeichnet, dass die starke Säurefunktion des Polyelektrolytmonomeren ausgewählt ist aus einer Sulfonsäuregruppe -SO₃H und einer Phosphonsäuregruppe, die teilweise oder vollständig neutralisiert sind, wobei weiter bevorzugte erfindungsgemäße Zusammensetzungen dadurch gekennzeichnet sind, dass das Polyelektrolytmonomer mit starker Säurefunktion ausgewählt ist aus 2-Methyl-2[(1-oxo-2-propenyl)amino]-1-propansulfonsäure, die teilweise oder vollständig neutralisiert ist.

Im Sinne der vorliegenden Erfindung sind alle Polyelektrolytmonomere, die mit einer schwachen oder starken Säuregruppe funktionalisiert sind, teilweise oder vollständig neutralisiert als Natrium-, Kalium-, Ammonium-, Ethanolamin- oder Aminosäure-Salz. Bevorzugte erfindungsgemäße Zusammensetzungen sind daher dadurch gekennzeichnet, dass die Polyelektrolytmonomere im polymeren Verdicker d), die mit einer schwachen oder starken Säuregruppe funktionalisiert sind, teilweise oder vollständig neutralisiert sind als Natrium-, Kalium-, Ammonium-, Ethanolamin- oder Aminosäure-Salz.

In einer weiteren bevorzugten erfindungsgemäßen Zusammensetzung dieser bevorzugten Ausführungsform ist das neutrale Polyelektrolytmonomer im polymeren Verdicker d) ausgewählt aus 2-Hydroxyethylacrylat, 2,3-Dihydroxypropylacrylat, 2-Hydroxyethylmethacrylat, 2,3-Dihydroxy-propylmethacrylat, den ethoxylierten Derivaten der vorstehend genannten Ester mit einem Molekulargewicht zwischen 400 und 1000 g/mol, Acrylamid, Alkylacrylamiden, wie beispielsweise Methylacrylamid, Ethylacrylamid, n-Propylacrylamid und Isopropylacrylamid, Dialkylamiden, wie beispielsweise Dimethylacrylamid, Diethylacrylamid, Di-n-propylacrylamid und Diisopropylacrylamid, sowie Polyvinylpyrrolidon. In bevorzugten erfindungsgemäßen Zusammensetzungen ist das neutrale Polyelektrolytmonomer ausgewählt aus 2-Hydroxyethylacrylat, 2,3-Dihydroxypropylacrylat, 2-Hydroxyethylmethacrylat, 2,3-Dihydroxypropylmethacrylat, den ethoxylierten Derivaten der genannten Ester mit einem Molekulargewicht zwischen 400 und 1000 g/mol, Acrylamid, Dimethylacrylamid, Diethylacrylamid, Di-n-propylacrylamid, Diisopropylacrylamid sowie Polyvinylpyrrolidon. Besonders bevorzugte neutrale Polyelektrolytmonomere sind ausgewählt aus 2-Hydroxy-ethylacrylat, Acrylamid, Dimethylacrylamid und Polyvinylpyrrolidon.

Vernetzte Copolymere aus 2-Methyl-2[(1-oxo-2-propenyl)amino]-1-propansulfonsäure (AMPS) und Hydroxyethylacrylat, in Form eines inversen, auto-inversiblen Latex sind kommerziell erhältlich, z. B. unter den Handelsnamen Simulgel^{®}NS, Simulgel^{®}I-NS 100, Simulgel^{®}FL und Sepiplus^{®} S von der Firma Seppic.

Weiterhin besonders bevorzugt sind teilweise oder vollständig neutralisierte, vernetzte Copolymere aus 2-Methyl-2[(1-oxo-2-propenyl)amino]-1-propansulfonsäure (AMPS) und Acrylamid, in Form eines inversen, auto-inversiblen Latex kommerziell erhältlich, z. B. unter dem Handelsnamen Simulgel^{®}600 von der Firma Seppic.

Weiterhin besonders bevorzugt sind teilweise oder vollständig neutralisierte, vernetzte Copolymere aus 2-Methyl-2[(1-oxo-2-propenyl)amino]-1-propansulfonsäure (AMPS) und Acrylsäure, in Form eines inversen, auto-inversiblen Latex kommerziell erhältlich, z. B. unter den Handelsnamen Simulgel^{®}EG und Simulgel^{®}EPG von der Firma Seppic.

Weiterhin besonders bevorzugt sind teilweise oder vollständig neutralisierte, vernetzte Copolymere aus 2-Methyl-2[(1-oxo-2-propenyl)amino]-1-propansulfonsäure (AMPS), Acrylsäure bzw. Natriumacrylat, und (als drittem Monomerbaustein) Dimethylacrylamid, in Form eines inversen, auto-inversiblen Latex kommerziell erhältlich, z. B. unter dem Handelsnamen Simulgel^{®}SMS 88 von der Firma Seppic.

Allen vorgenannten, bevorzugt verwendeten inversen Polymerlatices ist gemein, dass sie mindestens ein Öl, bevorzugt ausgewählt aus weißen Mineralölen, Squalan, Isohexadecan und (gegebenenfalls hydriertem) Polyisobuten, sowie mindestens einen ÖI-in-Wasser-Emulgator, ausgewählt aus den Ethylenoxid-Addukten von Sorbitanoleat, Ricinusöl, das gewünschtenfalls gehärtet ist, Sorbitanlaurat und Laurylalkohol, sowie weiterhin ausgewählt aus der Polyethylenoxid-freien Öl-in-Wasser-Emulgatorklasse der C6-C22-Fettalkohol-Glucoseether, insbesondere Caprinic Glucoside, Caprylic Glucoside, Capric Glucoside, Lauric Glucoside, Myristic Glucoside, Cetyl Glucoside, Stearyl Glucoside, Arachidyl Glucoside, Behenyl Glucoside, besonders bevorzugt Caprylic Glucoside und Capric Glucoside, enthalten. Demnach sind bevorzugte erfindungsgemäße Zusammensetzungen dieser bevorzugten Ausführungsform dadurch gekennzeichnet, dass die Ölphase des inversen Polymerlatex mindestens ein Öl, ausgewählt aus weißen Mineralölen, Squalan, Isohexadecan und (gegebenenfalls hydriertem) Polyisobuten, enthält. Weiter bevorzugte erfindungsgemäße Zusammensetzungen dieser bevorzugten Ausführungsform sind dadurch gekennzeichnet, dass der in dem inversen Polymerlatex enthaltene ÖI-in-Wasser-Emulgator ausgewählt ist aus den Ethylenoxid-Addukten von Sorbitanoleat, Ricinusöl, das gewünschtenfalls gehärtet ist, Sorbitanlaurat und Laurylalkohol, aus der Polyethylenoxid-freien ÖI-in-Wasser-Emulgatorklasse der C6-C22-Fettalkohol-Glucoseether, insbesondere Caprinic Glucoside, Caprylic Glucoside, Capric Glucoside, Lauric Glucoside, Myristic Glucoside, Cetyl Glucoside, Stearyl Glucoside, Arachidyl Glucoside, Behenyl Glucoside, besonders bevorzugt Caprylic Glucoside und Capric Glucoside.

Die erfindungsgemäß bevorzugten verdickenden Polymerlatices d) weisen vorzugsweise einen Polymergehalt von 30 - 90 Gew.-%, bevorzugt 35 - 75 Gew.-% und besonders bevorzugt 40 - 60 Gew.-%, jeweils bezogen auf den gesamten Latex, auf. Der Polymergehalt des Latex hat dabei jedoch eher nur eine Bedeutung für die Herstellung der erfindungsgemäßen Zusammensetzungen, z. B. in Bezug auf die Mischbarkeit bzw. Dosierbarkeit. Für die erfindungsgemäßen Zusammensetzungen selbst ist eher der Polymergehalt an sich, bezogen auf die erfindungsgemäße Zusammensetzung, bedeutsam.

Wird die erfindungsgemäße verdickte Zusammensetzung als Kosmetikum formuliert, wird - bezogen auf die gesamte erfindungsgemäße Zusammensetzung - das verdickende Polymer d) bevorzugt in Mengen von 0,1 - 5 Gew.-%, bevorzugt 0,3 - 3 Gew.-% und besonders bevorzugt 0,5 - 2,5 Gew.-%, bezogen auf die gesamte erfindungsgemäße kosmetische Zusammensetzung, eingesetzt, wobei Polymergehalte von 0,4, 0,6, 0,7, 0,8, 0,9, 1,0, 1,1, 1,2, 1,3, 1,4, 1,5, 1,6, 1,7, 1,8, 1,9, 2,0, 2,1, 2,2, 2,3 und 2,4 Gew.-% besonders bevorzugt und Polymergehalte von 0,3, 0,4, 0,5, 0,6, 0,7, 0,8, 0,9, 1,0 und 1,1 Gew.-% außerordentlich bevorzugt sein können.

Es hat sich überraschenderweise gezeigt, dass sich Betain (Trimethylammoniumacetat) besonders gut in die erfindungsgemäßen Mittel zur topischen Behandlung der Haut einarbeiten läßt und die erfindungsgemäße Wirkstoffkombination unterstützt, indem die hautberuhigenden und -pflegenden Effekte weiter verstärkt werden. Eine weitere erfindungsgemäß besonders bevorzugte Zusammensetzung ist daher dadurch gekennzeichnet, dass sie bezogen auf ihr Gewicht 0,5 bis 5 Gew.-%, vorzugsweise 1,0 bis 4 Gew.-%, besonders bevorzugt 1,5 bis 3 Gew.-% und insbesondere 1,75 bis 2,5 Gew.-% Trimethylammoniumacetat (H₃C)₃N⁺CH₂COO⁻ enthält.

Ein weiterer feuchtigkeitsspendender und hautweichmachender Wirkstoff, der hervorragend mit der erfindungsgemäßen Wirkstoffkombination harmoniert und seine Wirkung mit diesem schneller und nachhaltiger entfaltet, ist N,N'-Bis(2-hydroxyethyl)harnstoff. Zudem bewirkt die Anwesenheit dieses Inhaltsstoffes in erfindungsgemäßen Zusammensetzungen, dass die Wirksamkeit ausgewählter kosmetischer oder dermatologischer Wirkstoffe optimiert wird. Weitere erfindungsgemäß besonders bevorzugte Zusammensetzungen enthalten bezogen auf ihr Gewicht 0,25 bis 5 Gew.-%, vorzugsweise 0,5 bis 4 Gew.-%, besonders bevorzugt 0,75 bis 3 Gew.-% und insbesondere 1 bis 2 Gew.-% N,N'-Bis(2-hydroxyethyl)harnstoff.

Nachfolgend werden einige besonders bevorzugte Stoffe ausführlicher beschrieben - dabei wird überwiegend auf erfindungsgemäße Hautbehandlungsmittel Bezug genommen. Selbstverständlich können die genannten Stoffe auch in Haarbehandlungsmitteln, anderen kosmetischen mitteln oder den vorstehend genannten nicht-kosmetischen Mitteln wie Weichspülern oder WC-Reinigern enthalten sein. Insofern ist der nachfolgend verwendete Begriff "Hautbehandlungsmittel" nicht beschränkend zu verstehen.

Im folgenden werden bevorzugte Inhaltsstoffe der erfindungsgemäßen Mittel näher beschrieben. Anionische Tenside sind bevorzugt in den erfindungsgemäßen Mitteln enthalten. Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie z. B. aus C₁₂₋₁₈-monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse bzw. Neutralisation gewonnen werden. Ebenso sind auch die Ester von α-Sulfofettsäuren (Estersulfonate), z.B. die α-sulfonierten Methylester der hydrierten Kokos-, Palmkern- oder Talgfettsäuren, geeignet. Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁-Alkohole mit im Durchschnitt 3,5 Mol Ethylenoxid (EO) oder C₁₂₋₁₈-Fettalkohole mit 1 bis 4 EO, sind geeignet. Sie werden insbesondere in Reinigungsmitteln aufgrund ihres hohen Schaumverhaltens vorzugsweise nur in relativ geringen Mengen, beispielsweise in Mengen von 1 bis 5 Gew.-%, eingesetzt. Vorzugsweise können die erfindungsgemäßen Mittel frei von Schwefelsäuremonoester sein. Eine weitere Klasse von Aniontensiden ist die durch Umsetzung von Fettalkoholethoxylaten mit Natriumchloracetat in Gegenwart basischer Katalysatoren zugängliche Klasse der Ethercarbonsäuren. Sie haben die allgemeine Formel: R¹⁰ O-(CH₂-CH₂-O)*ₚ*-CH₂-COOH mit R¹⁰=C₁-C₁₈ und *p* = 0,1 bis 20. Ethercarbonsäuren sind wasserhärteunempfindlich und weisen ausgezeichnete Tensideigenschaften auf. Geeignete anionische Tenside sind beispielsweise auch die Partialester von Di- oder Polyhydroxyalkanen, Mono- und Disacchariden, Polyethylenglycolen mit den EO-Addukten von Maleinsäureanhydrid an mindestens einfach ungesättigte Carbonsäuren mit einer Kettenlänge von 10 bis 25 Kohlenstoffatomen mit einer Säurezahl von 10 bis 140.

Bevorzugte anionische Tenside weisen neben einem unverzweigten oder verzweigten, gesättigten oder ungesättigten, aliphatischen oder aromatischen, acyclischen oder cyclischen, optional alkoxylierten Alkylrest mit 4 bis 28, vorzugsweise 6 bis 20, insbesondere 8 bis 18, besonders bevorzugt 10 bis 16, äußerst bevorzugt 12 bis 14 Kohlenstoffatomen, zwei oder mehr anionische, insbesondere zwei, Säuregruppen, vorzugsweise Carboxylat-, Sulfonat- und/oder Sulfatgruppen, insbesondere eine Carboxylat- und eine Sulfatgruppe, auf. Beispiele dieser Verbindungen sind die alpha-Sulfofettsäuresalze, die Acylglutamate, die Monoglyceriddisulfate und die Alkylether des Glycerindisulfats sowie insbesondere monoveresterte Sulfosuccinate.

Der Gehalt des erfindungsgemäßen Mittels an anionischen Tensiden, vorzugsweise an den genannten anionischen Tensiden, kann in weiten Bereichen variieren, je nachdem welchem Zweck das betreffende Mittel dient. So kann ein erfindungsgemäßes Mittel sehr große Mengen Aniontensid enthalten, vorzugsweise bis zu einer Größenordnung von bis zu 40, 50 oder 60 Gew.- oder mehr. Ebenso kann ein erfindungsgemäßes Mittel nur sehr geringe Mengen Aniontensid enthalten, beispielsweise weniger als 15 oder 10 Gew.-% oder weniger als 5 Gew.-% oder noch weniger. Vorteilhafterweise sind in den erfindungsgemäßen Mitteln jedoch Aniontenside in Mengen von 0,1 bis 40 Gew.-% und insbesondere 5 bis 30 Gew.-% enthalten, wobei Konzentrationen oberhalb von 10 Gew.-% und sogar oberhalb von 15 Gew.-% besondere Bevorzugung finden. Nach einer bevorzugten Ausführungsform enthält das erfindungsgemäße Mittel anionische Tenside, vorzugsweise in Mengen von zumindest 0,01 Gew.-%, bezogen auf das gesamte Mittel. Nach einer anderen bevorzugten Ausführungsform kann das erfindungsgemäße Mittel frei von Aniontensid sein.

Zusätzlich zu den genannten anionischen Tensiden, aber auch unabhängig von diesen, können in den erfindungsgemäßen Mitteln Seifen enthalten sein. Geeignet sind insbesondere gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, z.B. Kokos-, Palmkern- oder Talgfettsäuren, abgeleitete Seifengemische. Der Gehalt des Mittels an Seifen beträgt, unabhängig von anderen Aniontensiden, vorzugsweise nicht mehr als 3 Gew.-% und insbesondere 0,5 bis 2,5 Gew.-%, bezogen auf das gesamte Mittel. Nach einer anderen bevorzugten Ausführungsform ist das erfindungsgemäße Mittel frei von Seife.

Die anionischen Tenside und Seifen können in Form ihrer Natrium-, Kalium- oder Ammoniumsalze sowie als lösliche Salze organischer Basen, wie Mono-, Di- oder Triethanol-amin, vorliegen. Vorzugsweise liegen sie in Form ihrer Natrium- oder Kaliumsalze, insbesondere in Form der Natriumsalze vor.

Vorteilhafterweise können nichtionische Tenside in den erfindungsgemäßen Mitteln ebenfalls enthalten sein, sowohl in festen wie in flüssigen Mitteln. Beispielsweise kann ihr Gehalt bis zu 2 oder 3 oder 5 Gew.-% betragen. Es können auch größere Mengen an nichtionischem Tensid enthalten sein, beispielsweise bis zu 5 Gew.-% oder 10 Gew.-% oder 15 Gew.-% oder 20 Gew.-%, 30 Gew.-%, 40 Gew.-%, 50 Gew.-% oder sogar darüber hinaus, falls es zweckmäßig ist. Sinnvolle Untergrenzen können bei Werten von 0,01, 0,1, 1, 2, 3 oder 4 Gew.-% liegen. Vorzugsweise sind die nichtionischen Tenside aber in größeren Mengen, also bis zu 50 Gew.-%, vorteilhafterweise von 0,1 bis 40 Gew.-%, besonders bevorzugt von 0,5 bis 30 und insbesondere von 2 bis 25 Gew.-%, jeweils bezogen auf das gesamte Mittel, enthalten. Nach einer bevorzugten Ausführungsform enthält das erfindungsgemäße Mittel nichtionische Tenside, vorzugsweise in Mengen von zumindest 0,1 Gew.-%, bezogen auf das gesamte Mittel. Nach einer anderen bevorzugten Ausführungsform kann das erfindungsgemäße Mittel frei von Niotensid sein. Vorteilhafterweise können alle aus dem Stand der Technik bekannten nichtionischen Tenside in den erfindungsgemäßen Mitteln enthalten sein.

Die erfindungsgemäßen Hautbehandlungsmittel können vorzugsweise auch mindestens ein kationisches Tensid enthalten. Geeignete Kationtenside sind beispielsweise oberflächenaktive quaternäre Verbindungen, insbesondere mit einer Ammonium-, Sulfonium-, Phosphonium-, Jodonium- oder Arsoniumgruppe. Durch den Einsatz von quaternären oberflächenaktiven Verbindungen mit antimikrobieller Wirkung kann das Mittel mit einer antimikrobiellen Wirkung ausgestaltet werden bzw. dessen gegebenenfalls aufgrund anderer Inhaltsstoffe bereits vorhandene antimikrobielle Wirkung verbessert werden.

Die erfindungsgemäßen Hautbehandlungsmittel können ein oder mehrere kationische Tenside enthalten, vorteilhafterweise in Mengen, bezogen auf die Gesamtzusammensetzung, von 0 bis 30 Gew.-%, noch vorteilhafter größer 0 bis 20 Gew.-%, vorzugsweise 0,01 bis 10 Gew.-%, insbesondere 0,1 bis 5 Gew.-%. Geeignete Mindestwerte können auch bei 0,5, 1, 2 oder 3 Gew.-% liegen. Nach einer bevorzugten Ausführungsform enthält das erfindungsgemäße Mittel kationische Tenside, vorzugsweise in Mengen von zumindest 0,1 Gew.-%, bezogen auf das gesamte Mittel. Nach einer anderen bevorzugten Ausführungsform kann das erfindungsgemäße Mittel frei von Kationtensid sein.

Ebenso können die erfindungsgemäßen Hautbehandlungsmittel auch mindestens ein amphoteres Tensid enthalten. Die erfindungsgemäßen Hautbehandlungsmittel können ein oder mehrere amphotere Tenside enthalten, vorteilhafterweise in Mengen, bezogen auf die Gesamtzusammensetzung, von 0 bis 30 Gew.-%, noch vorteilhafter größer 0 bis 20 Gew.-%, vorzugsweise 0,01 bis 10 Gew.-%, insbesondere 0,1 bis 5 Gew.-%. Nach einer anderen bevorzugten Ausführungsform kann das erfindungsgemäße Mittel frei von amphoteren Tensiden sein.

Nach einer bestimmten Ausführungsform können die erfindungsgemäßen Mittel nur sehr wenig Gesamttensid enthalten, z.B. kann die Gesamttensidmenge unter 20 Gew.-%, 15 Gew.-%, 10 Gew.-% oder 5 Gew.-%, vorteilhafterweise sogar unter 3 Gew.-% oder unter 1 Gew.-%, insbesondere sogar unter 0,5 Gew.-% oder unter 0,1 Gew.-% liegen, Gew.-% jeweils bezogen auf das gesamte Mittel. Vorzugsweise beträgt der Gesamttensidgehalt aber zumindest 0,01 Gew.-%, 0,1 Gew-% oder 1 Gew.-%, bezogen auf das gesamte Mittel.

Im Falle flüssiger Mittel enthält das erfindungsgemäße Mittel nach einer bevorzugten Ausführungsform Wasser in einer Menge von mehr als 20 Gew.-%, vorteilhafterweise mehr als 30 Gew.-%., in weiter vorteilhafter Weise mehr als 40 Gew.-%, noch vorteilhafter mehr als 50 Gew.-%, insbesondere 60 bis 99 Gew.-%, besonders bevorzugt 70 bis 98 Gew.-% und äußerst bevorzugt 80 bis 95 Gew.-%, bezogen auf das gesamte Mittel. Die Obergrenze an Wasser kann auch bei 80 Gew.-%, 70 Gew.-%, 60 Gew.-%, 50 Gew.-%, 40 Gew.-%, 30 Gew.-%, 20 Gew.-% oder 10 Gew.-% Gew.-% liegen, bezogen auf das gesamte Mittel.

Die Untergrenze an Wasser kann z.B. auch bei 80 Gew.-%, 70 Gew.-%, 60 Gew.-%, 50 Gew.-%, 40 Gew.-%, 30 Gew.-%, 20 Gew.-% oder 10 Gew.-% liegen, bezogen auf das gesamte Mittel.

Die genannten Ober- und Untergrenzen können natürlich sinnvoll kombiniert werden, z.B. zu Wassergehalten von 60-80 Gew.-% oder 10-30 Gew.-% usw.

Liegt das erfindungsgemäße Mittel in partikulärer Form vor, beispielsweise als Anti-Akne-Puder oder als mattierender Puder, so können die Partikel nachbehandelt werden, beispielsweise indem man die Partikel des Mittels verrundet.

In einer weiteren bevorzugten Ausführungsform enthält das erfindungsgemäße Hautbehandlungsmittel mindestens eine UV-absorbierende Substanz enthalten. Bei einem Einsatz in geringen Mengen (bis etwa 1 Gew.-%) dient die UV-absorbierende Substanz dazu, vorteilhafterweise die Lichtbeständigkeit sonstiger Rezepturbestandteile zu verbessern (Produktschutz). Bei einem Einsatz in höheren Mengen (ab mehr als 1 Gew.-%) dient die UV-absorbierende Substanz dazu, die behandelte Haut vor den schädlichen Auswirkungen der UV-Strahlung zu schützen. Unter UV-absorbierende Substanz sind organische und anorganische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimtsäurederivate), gegebenenfalls mit Cyanogruppen in 2-Stellung, Salicylsäureester sowie Naturstoffe wie Umbelliferon und die körpereigene Urocansäure geeignet.

Die mindestens eine UV-absorbierende Substanz ist in bevorzugten erfindungsgemäßen Hautbehandlungsmitteln in einer Gesamtmenge von 0,01 Gew.-% bis 20 Gew.-%, besonders bevorzugt 1 - 10 Gew.-%, außerordentlich bevorzugt 2 Gew.-% bis 7 Gew.-%, enthalten.

Bevorzugte erfindungsgemäße Mittel enthalten ein oder mehrere Lösungsmittel in einer Gesamtmenge von üblicherweise bis zu 90 Gew.-%, vorzugsweise 0,1 bis 30 Gew.-%, insbesondere 2 bis 20 Gew.-%, besonders bevorzugt 3 bis 15 Gew.-%, äußerst bevorzugt 5 bis 12 Gew.-%, beispielsweise 5,3 oder 10,6 Gew.-%, jeweils bezogen auf das gesamte Mittel.

Das erfindungsgemäße Mittel kann vorzugsweise einen oder mehrere antimikrobielle Wirkstoffe bzw. Konservierungsmittel in einer Menge von üblicherweise 0,0001 bis 3 Gew.-%, vorzugsweise 0,0001 bis 2 Gew.-%, insbesondere 0,0002 bis 1 Gew.-%, besonders bevorzugt 0,0002 bis 0,2 Gew.-%, äußerst bevorzugt 0,0003 bis 0,1 Gew.-%, enthalten.

Weitere bevorzugte erfindungsgemäße Zusammensetzungen sind dadurch gekennzeichnet, dass zur Verbesserung der Hautfeuchtigkeit unter Verzicht auf komedogene Substanzen mindestens ein alkyl- oder hydroxyalkylsubstituierten Harnstoff der allgemeinen Formel (A) enthalten ist, 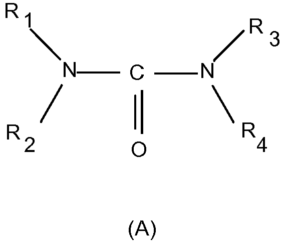 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.

Weitere bevorzugte erfindungsgemäße Zusammensetzungen sind dadurch gekennzeichnet, dass der mindestens eine alkyl- oder hydroxyalkylsubstituierte Harnstoff der allgemeinen Formel (A) ausgewählt ist aus Verbindungen, in denen R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ und R₂ und gleichzeitig mindestens einer der Reste R₃ und R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.

Weitere bevorzugte erfindungsgemäße Zusammensetzungen sind dadurch gekennzeichnet, dass der mindestens eine alkyl- oder hydroxyalkylsubstituierte Harnstoff der allgemeinen Formel (A) ausgewählt ist aus Bis-N,N'- (2-hydroxyethyl)harnstoff. Erfindungsgemäß besonders bevorzugte Zusammensetzungen sind dadurch gekennzeichnet, dass sie mindestens einen alkyl- oder hydroxyalkylsubstituierten Harnstoff gemäß Formel (A), insbesondere N,N'-Bis-(2-hydroxyethyl)-harnstoff, in einer Gesamtmenge von 0,01 - 50 Gew.-%, bevorzugt 0,1 - 20 Gew.-%, besonders bevorzugt 1 - 10 Gew.-% und außerordentlich bevorzugt 2 - 5 Gew.-%, bezogen auf die gesamte Zusammensetzung, enthalten. N,N'-Bis-(2-hydroxyethyl)harnstoff, bei Raumtemperatur ein kristalliner Feststoff, ist z. B. als Handelsprodukt Hydrovance der National Starch and Chemical Company als ca. 45 - 55 %ige wässrige Lösung mit einem Gehalt an Harnstoff und Ammoniumlactat erhältlich.

In einer bevorzugten Ausführungsform enthalten die erfindungsgemäßen Zusammensetzungen mindestens einen weiteren kosmetischen Wirkstoff, der ausgewählt ist aus Monomeren, Oligomeren und Polymeren von Aminosäuren, N-C₂-C₂₄-Acylaminosäuren und/oder den Estern und/oder den physiologisch verträglichen Metallsalzen dieser Substanzen, sowie Mischungen dieser Wirkstoffe.

Die Monomere der Aminosäuren und/oder der N-C₂-C₂₄-Acylaminosäuren sind ausgewählt aus Alanin, Arginin, Asparagin, Asparaginsäure, Canavanin, Citrullin, Cystein, Cystin, Desmosin, Dipalmitoylhydroxyprolin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, N-Acetyl-L-cystein, Zink- und Natriumpyroglutamat und Natriumlauroylglutamat.

Der C₂ - C₂₄-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten. Mit den vorgenannten C₂ - C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift DPHP von der Firma Seppic erhältlich ist.

Die physiologisch verträglichen Salze der erfindungsgemäß bevorzugten Wirkstoffe, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Unter Aminosäureoligomeren werden erfindungsgemäß Peptide mit 2 - 30, bevorzugt 2 - 15, Aminosäuren, verstanden. Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die N-acyliert und/oder verestert sein können. Zahlreiche dieser Aminosäureoligomere stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z.B. die Collagensynthese, induzieren, beziehungsweise sind in der Lage, an die Sequenz Arg-Phe-Lys in Thrombospondin I (TSP-1) zu binden und damit aktives TGF-ß (*tissue growth factor*), der die Synthese von Collagen in dermalen Fibroblasten induziert, freizusetzen. Derartige Aminosäureoligomere können als Wirkstoffe gegen die Hautalterung verwendet werden.

Die Polymere der Aminosäuren und/oder der N-C₂-C₂₄-Acylaminosäuren sind bevorzugt ausgewählt aus pflanzlichen und tierischen Proteinhydrolysaten und/oder Proteinen. Tierische Proteinhydrolysate sind z. B. Elastin-, Collagen-, Keratin-, Seiden-, Conchiolin- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate.

Weiterhin erfindungsgemäß bevorzugt sind Keratinhydrolysate, insbesondere Wollkeratinhydrolysate. Ein besonders bevorzugtes Wollkeratinhydrolysat ist unter der Bezeichnung Keratec Pep von der Firma Croda erhältlich. Keratec Pep weist eine kleinere Molekulargewichtsfraktion mit einem durchschnittlichen Molekulargewicht von 150 Dalton und eine größere Molekulargewichtsfraktion mit einem durchschnittlichen Molekulargewicht von 1265 Dalton auf. Weiterhin erfindungsgemäß bevorzugt sind Conchiolinhydrolysate, insbesondere solche, die unter den Bezeichnungen Pearl Protein Extract und Pearl Protein Extract BG von der Firma Maruzen erhältlich sind. Proteinhydrolysate können naturgemäß auch monomere Aminosäuren und Oligopeptide enthalten; ihre Zusammensetzung ist normalerweise nicht definiert. Ebenfalls bevorzugt ist der Einsatz von Acylderivaten der Proteinhydrolysate, z. B. in Form ihrer Fettsäure-Kondensationsprodukte. Erfindungsgemäß bevorzugt sind auch kationisierte Proteinhydrolysate. Besonders bevorzugt sind kationische Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Weiterhin können die kationischen Proteinhydrolysate auch noch weiter derivatisiert sein.

In einer weiteren bevorzugten Ausführungsform sind die Polymeren der Aminosäuren ausgewählt aus DNA-Reparaturenzymen. Erfindungsgemäß bevorzugte DNA-Reparaturenzyme sind Photolyase und T4 Endonuclease V, letztere im weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen. Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans,* einem phototrophen marinen Mikroorganismus. Die Photolyase aus *A. nidulans* wird in technisch relevanten Mengen mittlerweile aus E. coli gewonnen. Photolyase ist zur Aktivierung auf Licht angewiesen. Das Enzym T4 Endonuclease V wird vom denV-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv. Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosome™, liposomenverkapselte T4N5 z. B. unter der Bezeichnung Ultrasome™ von der Firma AGI Dermatics, USA, erhältlich. Erfindungsgemäß besonders bevorzugte Zusammensetzungen sind dadurch gekennzeichnet, dass sie mindestens eines der Handelsprodukte Photosomes™ oder Ultrasomes™ in Gesamtmengen von 0,1 - 10 Gew.-%, bevorzugt 0,5 - 5,0 Gew.-% und besonders bevorzugt 1,0 - 4,0 Gew.-%, bezogen auf die gesamte erfindungsgemäße Zusammensetzung, enthalten.

Erfindungsgemäß besonders bevorzugte Zusammensetzungen sind dadurch gekennzeichnet, dass sie mindestens ein Monomer, Oligomer oder Polymer von Aminosäuren, N-C₂-C₂₄-Acylaminosäuren und/oder den Estern und/ oder den physiologisch verträglichen Metallsalzen dieser Substanzen in Gesamtmengen von 0,0000001 - 10 Gew.-%, bevorzugt 0,001 - 5 Gew.-% und besonders bevorzugt 0,01 - 1 - 2 - 3 Gew.-%, jeweils bezogen auf den Aktivsubstanzgehalt in der gesamten erfindungsgemäßen Zusammensetzung, enthalten.

In einer weiteren bevorzugten Ausführungsform enthalten die erfindungsgemäßen Zusammensetzungen mindestens ein DNA-Oligonucleotid oder mindestens ein RNA-Oligonucleotid. Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glykosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5 '-triphosphat) und GTP (Guanosin-5'-triphosphat). Ein erfindungsgemäß besonders bevorzugtes Oligonucleotid ist das Thymidin-Dinucleotid.

Erfindungsgemäß besonders bevorzugte Zusammensetzungen sind dadurch gekennzeichnet, dass sie mindestens ein DNA-Oligonucleotid und/oder ein RNA-Oligonucleotid in Gesamtmengen von 0,000001 - 5 Gew.-%, bevorzugt 0,0001 - 0,5 Gew.-% und besonders bevorzugt 0,001 - 0,05 Gew.-%, bezogen auf die gesamte Zusammensetzung, enthalten.

In einer weiteren bevorzugten Ausführungsform enthalten die erfindungsgemäßen Zusammensetzungen mindestens eine natürliche Betainverbindung. Erfindungsgemäße natürliche Betainverbindungen sind natürlich vorkommende Verbindungen mit der Atomgruppierung R₃N⁺-CH₂-X-COO gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻) jeweils mit Me = Methyl und X = C-C-Einfachbindung (im Falle des Betains) oder X = -CHOH-CH₂- (im Falle des Carnitins). Erfindungsgemäß besonders bevorzugte Zusammensetzungen sind dadurch gekennzeichnet, dass sie mindestens eine natürliche Betainverbindung in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.

In einer weiteren bevorzugten Ausführungsform enthalten die erfindungsgemäßen Zusammensetzungen mindestens ein Vitamin, Provitamin oder eine als Vitaminvorstufe bezeichnete Verbindung aus den Vitamingruppen A, B, C, E, H und K und den Estern der vorgenannten Substanzen.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente erfindungsgemäß besonders bevorzugt sind Vitamin A-Säure und deren Ester, Vitamin A-Aldehyd und Vitamin A-Alkohol sowie dessen Ester, wie Retinylpalmitat und Retinylacetat. Erfindungsgemäß besonders bevorzugte Zusammensetzungen sind dadurch gekennzeichnet, dass sie mindestens ein Vitamin, Provitamin oder eine als Vitaminvorstufe bezeichnete Verbindung aus den Vitamingruppe A oder mindestens einen Ester hiervon in Gesamtmengen von 0,001 - 2 Gew.-%, bevorzugt 0,05 - 0,05 - 1 Gew.-%, bezogen auf die gesamte Zusammensetzung, enthalten.

Zur Vitamin B-Gruppe oder zu dem Vitamin B-Komplex gehören unter anderem Vitamin B₁, Trivialname Thiamin. Bevorzugt wird Thiaminhydrochlorid in Mengen von 0,0005 bis 0,1 - 1 Gew.-%, bezogen auf die gesamte erfindungsgemäße Zusammensetzung, eingesetzt; Vitamin B₂, Trivialname Riboflavin. Bevorzugt werden Riboflavin oder seine Derivate in Mengen von 0,0005 bis 0,1 - 1 Gew.-%, bezogen auf die gesamte erfindungsgemäße Zusammensetzung, eingesetzt; Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Zusammensetzungen bevorzugt in Mengen von 0,0005 bis 0,1 - 1 Gew.-%, bezogen auf die gesamte erfindungsgemäße Zusammensetzung, enthalten ist; Vitamin B₅ (Pantothensäure und Panthenol). Bevorzugt wird Panthenol eingesetzt. Erfindungsgemäß bevorzugte Derivate des Panthenols sind insbesondere die Ester und Ether des Panthenols sowie kationisch derivatisierte Panthenole. In einer weiteren bevorzugten Ausführungsform der Erfindung werden an Stelle von sowie zusätzlich zu Pantothensäure oder Panthenol auch Derivate des 2-Furanon mit der allgemeinen Strukturformel (VIT-I) eingesetzt.

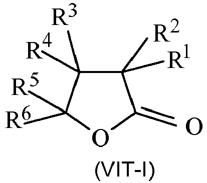

Besonders bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Ein bevorzugtes Derivat ist die Substanz Dihydro-3-hydroxy-4,4-dimethyl-2(3H)-furanon mit dem Trivialnamen Pantolacton (Merck). Das erfindungsgemäß außerordentlich bevorzugte 2-Furanon-Derivat ist Pantolacton (Dihydro-3-hydroxy-4,4-dimethyl-2(3H)-furanon), wobei in Formel (I) R₁ für eine Hydroxylgruppe, R² für ein Wasserstoffatom, R³ und R₄ für eine Methylgruppe und R⁵ und R⁶ für ein Wasserstoffatom stehen. Das Stereoisomer (R)-Pantolacton entsteht beim Abbau von Pantothensäure.

Erfindungsgemäß besonders bevorzugte Zusammensetzungen sind dadurch gekennzeichnet, dass sie mindestens eine der genannten Verbindungen des Vitamin B₅-Typs sowie der 2-Furanon-derivate in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten; Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Erfindungsgemäß besonders bevorzugte Zusammensetzungen sind dadurch gekennzeichnet, dass sie mindestens eine Vitamin B₆-Komponente in einer Gesamtmenge von 0,0001 bis 1,0 Gew.-%, insbesondere in Mengen von 0,001 bis 0,01 Gew.-%, enthalten; Vitamin B₇ (Biotin), auch als Vitamin H oder "Hautvitamin" bezeichnet. Erfindungsgemäß besonders bevorzugte Zusammensetzungen sind dadurch gekennzeichnet, dass sie mindestens eine Komponente, ausgewählt aus Biotin und den Biotinestern, in einer Gesamtmenge von 0,0001 bis 1,0 Gew.-%, insbesondere 0,001 bis 0,01 Gew.-%, enthalten; Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Erfindungsgemäß besonders bevorzugte Zusammensetzungen sind dadurch gekennzeichnet, dass sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Zusammensetzung, enthalten; Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind dadurch gekennzeichnet, dass sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Zusammensetzung, enthalten.

In einer weiteren bevorzugten Ausführungsform enthalten die erfindungsgemäßen Zusammensetzungen mindestens eine Substanz, die ausgewählt ist aus den Vitaminen, Provitaminen und Vitaminvorstufen der Gruppe B₁, B₂, B₃, B₆, B₇, B₉, B₁₃ und deren Estern und aus Pantolacton. Bevorzugte Vitamine, Provitamine und Vitaminvorstufen der Gruppe C und deren Ester sind Vitamin C (Ascorbinsäure) und die Derivate Ascorbylpalmitat, -stearat, -dipalmitat, -acetat, Magnesiumascorbylphosphat, Natriumascorbylphosphat, Natrium- und Magnesiumascorbat, Dinatriumascorbylphosphat und -sulfat, Kaliumascorbyltocopherylphosphat, Chitosanascorbat oder Ascorbylglucosid. Die Kombination mit Tocopherolen kann ebenfalls bevorzugt sein. Erfindungsgemäß besonders bevorzugte Zusammensetzungen sind dadurch gekennzeichnet, dass sie mindestens eine der genannten Verbindungen des Vitamin C-Typs in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Zusammensetzung, enthalten.

Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Erfindungsgemäß besonders bevorzugte Zusammensetzungen sind dadurch gekennzeichnet, dass sie mindestens eine Substanz, ausgewählt aus Tocopherol und seinen Derivaten, in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Zusammensetzung, enthalten.

Vitamin A-palmitat (Retinylpalmitat), Pantolacton, Nicotinsäureamid, Pyridoxin, Pyridoxamin, Pyridoxal, Biotin, Ascorbylpalmitat, Ascorbylacetat, Mg-Ascorbylphosphat, Na-Ascorbylphosphat, Natrium- und Magnesiumascorbat und die Tocopherolester, besonders Tocopherylacetat, sind erfindungsgemäß besonders bevorzugt.

In einer weiteren bevorzugten Ausführungsform enthalten die erfindungsgemäßen Zusammensetzungen neben der erfindungsgemäßen Wirkstoffkombination mindestens eine α-Hydroxycarbonsäure, α-Ketocarbonsäure oder β-Hydroxycarbonsäure oder deren Ester-, Lacton- oder Salzform. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Erfindungsgemäß besonders bevorzugte Zusammensetzungen sind dadurch gekennzeichnet, dass sie neben der erfindungsgemäßen Wirkstoffkombination mindestens eine α-Hydroxycarbonsäure, α-Ketocarbonsäure und/oder β-Hydroxycarbonsäure oder ein Derivat, insbesondere einen Ester, ein Lacton oder ein Salz hiervon, in einer Gesamtmenge von 0,01 - 10 Gew.-%, bevorzugt 0,1 - 5 Gew.-%, besonders bevorzugt 0,5 - 1 - 2 Gew.-%, jeweils bezogen auf die gesamte Zusammensetzung, enthalten.

In einer weiteren bevorzugten Ausführungsform enthalten die erfindungsgemäßen Zusammensetzungen mindestens ein Flavonoid oder mindestens einen Flavonoid-reichen Pflanzenextrakt.

Erfindungsgemäß besonders bevorzugte Zusammensetzungen sind dadurch gekennzeichnet, dass sie mindestens ein Flavonoid in einer Gesamtmenge von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, enthalten.

In einer weiteren bevorzugten Ausführungsform enthalten die erfindungsgemäßen Zusammensetzungen mindestens ein Isoflavonoid oder mindestens einen Isoflavonoid-reichen Pflanzenextrakt. Zu den Isoflavonoiden werden an dieser Stelle die Isoflavone und die Isoflavon-Glycoside gezählt. Erfindungsgemäß besonders bevorzugte Zusammensetzungen sind dadurch gekennzeichnet, dass sie mindestens ein Isoflavonoid in einer Gesamtmenge von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten kosmetischen Zusammensetzung, enthalten.

In einer weiteren bevorzugten Ausführungsform enthalten die erfindungsgemäßen Zusammensetzungen mindestens ein Polyphenol oder einen Polyphenol-reichen Pflanzenextrakt. Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättern von Grünem Tee (camellia sinensis) und Mate. Ebenfalls besonders bevorzugt sind die Tannine.

Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay L, ein Extrakt aus den Kernen der Chardonnay-Traube, erhältlich von der Firma Croda.

Erfindungsgemäß bevorzugt werden die Polyphenole in Mengen von 0,001 bis 10 Gew.-%, besonders bevorzugt 0,005 bis 5 Gew.-% und außerordentlich bevorzugt 0,01 bis 3 Gew.-%, jeweils bezogen auf das Gewicht des Handelsproduktes, das mindestens ein Polyphenol enthält, in der gesamten erfindungsgemäßen Zusammensetzung, eingesetzt.

In einer weiteren bevorzugten Ausführungsform enthalten die erfindungsgemäßen Zusammensetzungen mindestens ein Ubichinon oder ein Ubichinol oder deren Derivate. Ubichinole sind die reduzierte Form der Ubichinone. Die erfindungsgemäß bevorzugten Ubichinone weisen die Formel (UBI-I) auf: 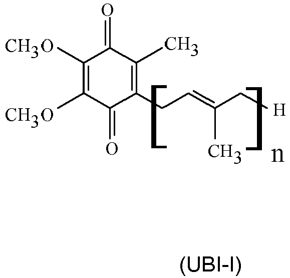 mit n = 6, 7, 8, 9 oder 10.

Besonders bevorzugt ist das Ubichinon der Formel (II) mit n = 10, auch bekannt als Coenzym Q10. Erfindungsgemäß besonders bevorzugte Zusammensetzungen sind dadurch gekennzeichnet, dass sie mindestens ein Ubichinon, Ubichinol oder ein Derivat hiervon in einer Gesamtmenge von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens ein natürlich vorkommendes Xanthin-Derivat, ausgewählt aus Coffein, Theophyllin, Theobromin und Aminophyllin. Erfindungsgemäß bevorzugt sind die natürlich vorkommenden Xanthin-Derivate in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß bevorzugt ist Ectoin in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen Kreatin. Kreatin ist der Trivialname für *N*-Methyl-guanidino-essigsäure bzw. *N*-Amidinosar-kosin. Erfindungsgemäß bevorzugt ist Kreatin in Mengen von 0,0001 bis 1 Gew.-%, besonders bevorzugt 0,001 bis 0,5 Gew.-% und außerordentlich bevorzugt 0,01 bis 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens einen Olivenblattextrakt (Olea Europaea (Olive) Leaf Extract). Erfindungsgemäß besonders bevorzugte Zusammensetzungen sind dadurch gekennzeichnet, dass sie mindestens einen Olivenblattextrakt in einer Gesamtmenge von 0,01 bis 5 Gew.-%, bevorzugt 0,1 bis 3 Gew.-% und besonders bevorzugt 0,5 bis 1 - 2 Gew.-%, jeweils bezogen auf den Extrakt als Handelsprodukt tel quel in der gesamten erfindungsgemäßen Zusammensetzung, enthalten. Olivenblattextrakte können einen hohen Gehalt an Oleanolsäure und/oder Oleanol aufweisen. In einer weiteren bevorzugten Ausführungsform enthalten die erfindungsgemäßen Zusammensetzungen Oleanolsäure, Oleanol und/oder Oleuropein. Erfindungsgemäß besonders bevorzugte Zusammensetzungen sind dadurch gekennzeichnet, dass sie Oleanolsäure, Oleanol und/oder Oleuropein in einer Gesamtmenge von 0,00001 bis 2 Gew.-%, bevorzugt 0,001 bis 1 Gew.-% und besonders bevorzugt 0,05 bis 0,1 Gew.-%, jeweils bezogen auf die gesamte erfindungsgemäße Zusammensetzung, enthalten.

In einer weiteren bevorzugten Ausführungsform enthalten die erfindungsgemäßen Zusammensetzungen Ursolsäure. Erfindungsgemäß besonders bevorzugte Zusammensetzungen sind dadurch gekennzeichnet, dass sie Ursolsäure in einer Gesamtmenge von 0,00001 bis 2 Gew.-%, bevorzugt 0,001 bis 1 Gew.-% und besonders bevorzugt 0,05 bis 0,1 Gew.-%, jeweils bezogen auf die gesamte erfindungsgemäße Zusammensetzung, enthalten.

In einer weiteren bevorzugten Ausführungsform enthalten die erfindungsgemäßen Zusammensetzungen mindestens einen Wirkstoff, der ausgewählt ist aus den Mono- und Polyhydroxystilbenen und deren Estern. Unter Polyhydroxystilbenen werden erfindungsgemäß Stilbene verstanden, die mit 2, 3, 4, 5, 6, 7, 8, 9 oder 10 Hydroxygruppen an den beiden Phenylresten substituiert sind, wobei diese verestert sein können. Mono- und Polyhydroxystilbene und deren Ester erhöhen und/oder verbessern die Interaktion zwischen der extrazellulären Matrix und den Fibroblasten. Erfindungsgemäß besonders bevorzugte Hydroxystilbene und deren Ester sind ausgewählt aus Resveratrol (trans-Stilben-3,4'-5-triol), den Resveratrolmono-, -di- und -triphosphorsäureestern und deren Salzen. Ein erfindungsgemäß besonders bevorzugter Resveratrolphosphorsäureester ist Trisodium Resveratrol Triphosphate, z. B. erhältlich von Ajinomoto.

Erfindungsgemäß besonders bevorzugte kosmetische oder dermatologische Zusammensetzungen sind dadurch gekennzeichnet, dass sie mindestens einen Wirkstoff, der ausgewählt ist aus den Mono- und Polyhydroxystilbenen und deren Estern, in einer Gesamtmenge von 0,000001 - 5 Gew.-%, bevorzugt 0,00001 - 1 Gew.-%, besonders bevorzugt 0,0001 - 0,1 Gew.-% und außerordentlich bevorzugt 0,005 - 0,05 Gew.-%, enthalten, jeweils bezogen auf den Gehalt an Aktivsubstanz in der gesamten Zusammensetzung.

In einer weiteren bevorzugten Ausführungsform enthalten die erfindungsgemäßen Zusammensetzungen mindestens einen Extrakt aus Apfelkernextrakten (Pyrus Malus (Apple) Fruit Extract). Derartige Extrakte erhöhen und/oder verbessern die Interaktion zwischen der extrazellulären Matrix und den Fibroblasten. Erfindungsgemäß besonders bevorzugte Apfelkernextrakte sind unter der Handelsbezeichnung Ederline von der Firma Seporga erhältlich. Das Produkt Ederline enthält Phytohormone, Isoflavonoide, Phytosterole, Triterpenoide, Tocopherole und natürliche Wachse. Ederline ist einmal in wasserlöslicher Form als Ederline-H (INCI: PEG-40 Hydrogenated Castor Oil, PPG-2-Ceteareth-9, Pyrus Malus (Apple) Fruit Extract), zum anderen in fettlöslicher Form als Ederline-L (INCI: Hexyldecanol, Pyrus Malus (Apple) Fruit Extract) erhältlich.

Erfindungsgemäß besonders bevorzugte kosmetische oder dermatologische Zusammensetzungen sind dadurch gekennzeichnet, dass sie den Rohstoff Ederline in Mengen von 0,1 - 10 Gew.-%, bevorzugt 1 - 8 Gew.-% und besonders bevorzugt 3 - 5 Gew.-%, jeweils bezogen auf die gesamte Zusammensetzung, enthalten. Erfindungsgemäß besonders bevorzugte kosmetische oder dermatologische Zusammensetzungen enthalten mindestens einen Apfelkernextrakt in Mengen von 0,00001 - 2 Gew.-%, bevorzugt 0,001 - 1,6 Gew.-% und besonders bevorzugt 0,03 - 1 Gew.-%, jeweils bezogen auf den Gehalt an Aktivsubstanz in der gesamten Zusammensetzung.

In einer weiteren bevorzugten Ausführungsform enthalten die erfindungsgemäßen Zusammensetzungen zur Unterstützung der Wirkung der erfindungsgemäßen Wirkstoffkombination mindestens einen sebumregulierenden Wirkstoff. Erfindungsgemäß bevorzugte sebumregulierende Wirkstoffe sind ausgewählt aus 10-Hydroxydecansäure, Sebacinsäure, Azelainsäure und den Estern der Azelainsäure, insbesondere Kaliumazeloyldiglycinat, 1,10-Decandiol und mindestens einem Extrakt aus Spiraea Ulmaria, Glycyrrhizin, das auch als Glycyrrhizinsäure oder Glycyrrhetinsäureglycosid bezeichnet wird und das 2-beta-Glucuronido-alpha-glucuronid der Glycyrrhetinsäure darstellt, sowie deren Salzen, Gerbsäure (tannic acid) und deren Salzen, Gallotanninen, Naringin, Mischungen aus Glycyrrhizin(salzen), Gerbsäure(salzen) und/oder Gallotanninen und Naringin, wie sie zum Beispiel als Handelsprodukt BiSCos Glynarin PF (INCI: Aqua (Water), Alcohol, Phenoxyethanol, Ammonium Glycyrrhizate, Tannic Acid, Naringine) von der Firma Biesterfeld erhältlich sind, weiterhin aus wasser- und öllöslichen Extrakten aus Hamamelis, Klettenwurzel und Brennessel, Zimtbaumextract (z. B. Sepicontrol^{®} A5 von der Firma Seppic), Chrysanthemenextrakt (z. B. Laricyl^{®} von Laboratoires Serobiologiques), Hefeproteinhydrolysaten, wie sie z.B. in den Handelsprodukten der Asebiol^{®}-Serie von Laboratoires Serobiologiques erhältlich sind, insbesondere Asebiol^{®} LS 2539 BT 2 (INCI: Aqua, Hydrolyzed Yeast Protein, Pyridoxine, Niacinamide, Glycerin, Panthenol, Allantoin, Biotin) und Asebiol^{®} LS 2539 BT (Aqua, Hydrolyzed Yeast Protein, Pyridoxine, Niacinamide, Glycerin, Panthenol, Propylene Glycol, Allantoin, Disodium Azelate, Biotin) und PEG-8 Isolauryl Thioether, wie es z. B. in dem Handelsprodukt "Antifettfaktor^{®} COS-218/2-A" von Cosmetochem enthalten ist (INCI: Aqua, Cetyl-PCA, PEG-8 Isolauryl Thioether, PCA, Cetyl Alcohol), sowie Mischungen der vorgenannten Substanzen. Bevorzugte Mischungen sind beispielsweise erhältlich als Handelsprodukt Acnacidol PG (Propylene Glycol, 10-Hydroxydecanoic acid, Sebacic acid, 1,10-Decandiol) von Vincience erhältlich. Ein bevorzugter Extrakt aus Spiraea Ulmaria ist z. B. im Produkt Seboregul 2 der Firma Silab enthalten. Kaliumazeloyldiglycinat ist z. B. in dem Produkt Azeloglicina der Firma Sinerga enthalten. Erfindungsgemäß besonders bevorzugte kosmetische oder dermatologische Zusammensetzungen sind dadurch gekennzeichnet, dass sie mindestens einen sebumregulierenden Wirkstoff in Gesamtmengen von 0,00001 bis 10 Gew.-%, bevorzugt 0,01 bis 5 Gew.-% und besonders bevorzugt 0,1 bis 1 -2 Gew.-%, jeweils bezogen auf Aktivsubstanz in der gesamten erfindungsgemäßen Zusammensetzung, enthalten.

Zur Unterstützung der erfindungsgemäßen Wirkstoffkombination enthalten die erfindungsgemäßen Hautbehandlungsmittel bevorzugt weiterhin mindestens einen partikelförmigen, sebumsorbierenden Wirkstoff, insbesondere Talkum, Stärke und andere Partikel. Erfindungsgemäß bevorzugte kosmetische oder dermatologische Zusammensetzungen sind dadurch gekennzeichnet, dass der partikelförmige, sebumsorbierende Wirkstoff ausgewählt ist aus anorganischen und organischen kosmetischen Adsorbentien mit mittleren Partikeldurchmessern von 0,1 - 100 µm, bevorzugt 0,5 - 50 µm, besonders bevorzugt 5 - 30 µm und außerordentlich bevorzugt 10 - 25 µm. Besonders bevorzugte anorganische Adsorbentien sind ausgewählt aus Kieselsäuren, insbesondere Aerosil^{®}-Typen, Kieselgelen, Siliciumdioxid, Tonen und Schichtsilicaten, insbesondere Bentoniten oder Kaolin, Magnesiumaluminiumsilikaten, insbesondere Talkum, und Bornitrid, sowie Mischungen der genannten Substanzen. Besonders bevorzugte organische Adsorbentien sind ausgewählt aus Stärken und Stärkederivaten, die chemisch und/oder physikalisch modifiziert sein können, insbesondere modifizierten Stärkederivaten vom Typ DRY FLO^{®} von Akzo Nobel, Cellulosepulvern, Lactoglobulinderivaten, insbesondere Natrium-C₈₋₁₆-Isoalkylsuccinyllactoglobulin-sulfonat, z. B. als Handelsprodukt Biopol^{®} OE von Brooks Industries erhältlich, Polymerpulvern aus Polyolefinen, Polycarbonaten, Polyurethanen, Polyamiden, Polyestern, Polystyrolen, Polyacrylaten, Polymethacrylaten, Polymethylmethacrylaten, (Meth)acrylat- oder (Meth)acrylat-Vinyliden-Copolymeren, Teflon oder Siliconen, wobei die genannten Polymerpulver in einer besonders bevorzugten Ausführungsform der Erfindung vernetzt sein können, sowie Mischungen der genannten Substanzen.

Polymerpulver auf Basis eines Polymethacrylat-Copolymers sind z. B. als Handelsprodukt Polytrap^{®} 6603 (Dow Corning) erhältlich. Andere Polymerpulver, z. B. auf Basis von Polyamiden, sind unter der Bezeichnung Orgasol^{®} 1002 (Polyamid-6) und Orgasol^{®} 2002 (Polyamid-12) von Elf Atochem erhältlich. Weitere erfindungsgemäß besonders bevorzugte Polymerpulver sind vernetzte Polymethacrylate (Micropearl^{®} M von SEPPIC oder Plastic Powder A von NIKKOL), vernetzte Polymethylmethacrylate (Micropearl^{®} M 305 und Micropearl^{®} M 310 von SEPPIC), Styrol-Divinylbenzol-Copolymere (z. B. Plastic Powder FP von NIKKOL), Polyethylen- und Polypropylen-Pulver (z. B. ACCUREL^{®} EP 400 von AKZO) oder auch Siliconpolymere (z. B. Silicone Powder X2-1605 von Dow Corning).

In einer weiteren bevorzugten Ausführungsform der Erfindung weist der partikelförmige, sebumsorbierende Wirkstoff Hohlräume auf. In einer weiteren bevorzugten Ausführungsform der Erfindung weist der partikelförmige, sebumsorbierende Wirkstoff halbkugelförmige Partikel auf, besonders bevorzugt sind mindestens 50 Gew.-% der Partikel halbkugelförmig. In einer weiteren bevorzugten Ausführungsform der Erfindung weist der partikelförmige, sebumsorbierende Wirkstoff halbkugelförmige Partikel mit Hohlräumen auf, besonders bevorzugt sind mindestens 50 Gew.-% der Partikel halbkugelförmig mit Hohlräumen.

Besonders bevorzugte partikelförmige, sebumsorbierende Wirkstoffe sind vernetzte Polymethylmethacrylate mit mittleren Partikeldurchmessern von 5 - 50 µm, insbesondere die Handelsprodukte Micropearl^{®} M 305 und Micropearl^{®} M 310 von SEPPIC. Außerordentlich bevorzugte partikelförmige, sebumsorbierende Wirkstoffe sind vernetzte Polymethylmethacrylate mit mittleren Partikeldurchmessern von 5 - 50 µm, die halbkugelförmige Partikel mit Hohlräumen umfassen, wobei besonders bevorzugt mindestens 50 Gew.-% der Partikel halbkugelförmig mit Hohlräumen sind, wie insbesondere das Handelsprodukt Micropearl^{®} M 310 von SEPPIC. Erfindungsgemäß besonders bevorzugte Zusammensetzungen sind dadurch gekennzeichnet, dass sie mindestens einen partikelförmigen sebumsorbierenden Wirkstoff in einer Gesamtmenge von 0,5 bis 10 Gew.-%, bevorzugt 1 bis 5 Gew.-% und besonders bevorzugt 1,5 bis 3 Gew.-%, jeweils bezogen auf die gesamte Zusammensetzung, enthalten.

Zur Unterstützung der erfindungsgemäßen Wirkstoffkombination enthalten die erfindungsgemäßen Hautbehandlungsmittel bevorzugt weiterhin mindestens einen adstringierenden Wirkstoff, der zu einer temporären Verengung der Hautporen führt, beispielsweise Tannine bzw. Gerbstoffe bzw. allgemein Polyphenole, weiterhin Aluminiumsalze, wie sie auch als Antitranspirant-Wirkstoffe Verwendung finden. Diese Wirkstoffe regulieren die Sebummenge an der Hautoberfläche durch Verzögerung des Sebumaustritts.

Zur Unterstützung der erfindungsgemäßen Wirkstoffkombination enthalten die erfindungsgemäßen Hautbehandlungsmittel bevorzugt weiterhin mindestens einen sebumregulierenden Wirkstoff, der das an der Aktivität der Talgdrüsen beteiligte Enzym 5-alpha-Reduktase inhibiert. Bevorzugte Inhibitoren der 5-alpha-Reduktase sind ausgewählt aus Finasterid^{®}, 4-Androsten-3-on-17β-carbonsäure, (4R)-5,10-seco-19-Norpregna-4,5-dien-3,10,20-trian, (5-alpha,20-R)-4-Diazo-21-hydroxy-20-methylpregnan-3-on, 4-Azasteroide, insbesondere 17β-N,N-diethylcarbamoyl-4-methyl-4-aza-5-alpha-androstan-3-on, 17-alpha-acetoxy-6-methylenpregn-4-en-3,20-dion, 3-Carboxy-androsta-3,5-dien-Steroid-Derivate und 3-Carboxy A Ring-Aryl-Steroide, 3-Androsten-3-carbonsäuren, 3-Carboxy-17β-substituierte Steroide, 17β-(N-t-butylcarboxamid)-5-a-androst-1-en-4-aza-3-on, 17β-(N-t-butylcarboxamid)-androst-3,5-dien-3-carbonsäure, 17β-(N,N-diisopropylcarboxamid)-androst-3,5-dien-3-carbonsäure, 17β-(N,N-diisopropylcarboxamid)-estra-1,3, 5(10)-trien-3-carbonsäure, 17β-(N-t-butylcarboxamid)-estra-1,3,5(10)-trien-3-carbonsäure, 17β-(N,N-diisopropylcarboxamid)-estra-1,3,5(10)-trien-3-sulfonsäure, 17β-(N-t-butylcarboxamid)-estra-1,3,5(10)-trien-3-sulfonsäure, 17β-(N,N-diisopropylcarboxamid)-estra-1,3,5(10)-trien-3-phosphonsäure, 17β-(N-t-butylcarboxamid)-estra-1,3,5(10)-trien-3-phosphonsäure, 17β-(N,N-diisopropylcarboxamid)-estra-1,3,5(10)-trien-3-phosphinsäure, 17β-(N-t-butylcarboxamid)-estra-1,3,5(10)-trien-3-phosphinsäure, 17β-(N-t-butylcarboxamid)-androst-3,5-dien-3-phosphinsäure, 17β-(N,N-diisopropylcarboxamid)-androst-3,5-dien-3-phosphinsäure, 17β-(N-t-butylcarboxamid)-androst-3,5-dien-3-phosphonsäure, (Z)-17β-(N,N-diisopropylcarboxamid)-androst-4-en-3-yliden-essigsäure, 17β-(N,N-diisopropylcarboxamid)-5a-androst-2-en-3-essigsäure, (Z)-17β-(N,N-diiso-propylcarboxamid)-5α-androst-3-yliden-essigsäure, 17β-(N,N-diisopropylcarboxamid)-5a-androst-3-en-3-essigsäure und 17β-(N-t-butylcarboxamid)-5a-androst-2-en-3-essigsäure sowie den physiologisch verträglichen Salzen der vorgenannten Säuren. Erfindungsgemäß besonders bevorzugte Zusammensetzungen sind dadurch gekennzeichnet, dass sie mindestens einen Inhibitor der 5-alpha-Reduktase in einer Gesamtmenge von 0,0001 bis 1 Gew.-%, bevorzugt 0,001 bis 0,5 Gew.-% und besonders bevorzugt 0,01 bis 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens einen Desoxyzucker oder ein mindestens einen Desoxyzucker-Baustein enthaltendes Polysaccharid. Erfindungsgemäß bevorzugte Desoxyzucker sind ausgewählt aus Rhamnose und Fucose. Ein erfindungsgemäß besonders bevorzugtes, Desoxyzucker-Bausteine enthaltendes Polysaccharid ist das Handelsprodukt Fucogel^{®} von Solabia mit der INCI-Bezeichnung Biosaccharide Gum-1. Ein weiteres erfindungsgemäß besonders bevorzugtes, Desoxyzucker-Bausteine enthaltendes Polysaccharid ist das Handelsprodukt Rhamnosoft^{®} von Solabia mit der INCI-Bezeichnung Biosaccharide Gum-2. Ein erfindungsgemäß besonders bevorzugtes, Desoxyzucker-Bausteine enthaltendes Polysaccharid ist das Handelsprodukt Fucogenol^{®} von Solabia mit der INCI-Bezeichnung Biosaccharide Gum-3. Ein erfindungsgemäß besonders bevorzugtes, Desoxyzucker-Bausteine enthaltendes Polysaccharid ist das Handelsprodukt Glycofilm^{®} von Solabia mit der INCI-Bezeichnung Biosaccharide Gum-4. Erfindungsgemäß bevorzugt sind weiterhin Mischungen der vorgenannten, mindestens einen Desoxyzucker-Baustein enthaltenden Polysaccharide, beispielsweise die Mischung aus Biosaccharide Gum-2 und Biosaccharide Gum-3, erhältlich als Handelsprodukt Elastinol plus^{®} von Solabia.

Erfindungsgemäß besonders bevorzugte kosmetische oder dermatologische Zusammensetzungen sind dadurch gekennzeichnet, dass sie mindestens einen Desoxyzucker und/ oder mindestens ein Desoxyzucker-Bausteine enthaltendes Polysaccharid in Gesamtmengen von 0,001 bis 5 Gew.-%, bevorzugt 0,01 bis 2 Gew.-% und besonders bevorzugt 0,1 bis 1 Gew.-%, jeweils bezogen auf die gesamte erfindungsgemäße Zusammensetzung, enthalten.

Vorteilhafterweise liegen die erfindungsgemäßen Hautbehandlungsmittel in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

In einer besonderen Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die sogenannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl-Emulsionen umwandeln. Bei anschließendem Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

Nach einer weiteren bevorzugten Ausführungsform ist das erfindungsgemäße Mittel frei von Parfümöl-Komponenten, insbesondere in Produkten für sensitive Haut.

Ein weiterer Gegenstand der vorliegenden Erfindung ist ein nicht-therapeutisches Verfahren zur nicht-therapeutischen, kosmetischen Behandlung unreiner Haut, dadurch gekennzeichnet, dass eine erfindungsgemäße kosmetische oder dermatologische topische Zusammensetzung auf die Haut aufgetragen wird.

Bezüglich weiterer bevorzugter Ausführungsformen der erfindungsgemäßen Verfahren gilt mutatis mutandis das zu den erfindungsgemäßen Mitteln Gesagte.

Erfindungsgemäßes Hautbehandlungsmittel (Angaben in Gew.-%)

| | |
|---|---|
| 1,6-Hexandiol | 8,000 |
| Betain | 2,000 |
| Dehydroxanthan Gum | 2,500 |
| N,N'-Bis(2-hydroxyethyl)harnstoff | 4,000 |
| Zinkgluconat | 0,200 |
| Urea | 0,250 |
| Sodium Benzoate | 0,500 |
| Sodium Salicylate | 0,500 |
| 1,10-Decanediol | 0,300 |
| 10-Hydroxydecanoic Acid | 0,300 |
| Sebacic Acid | 0,300 |
| Sodium Acrylate/Sodium Acryloyldimethyl Taurate Copolymer | 0,300 |
| Ammonium Acryloyldimethyltaurate/VP Copolymer | 1,000 |
| Citric Acid | 0,500 |
| Polyisobutene | 0,700 |
| Sorbitan Oleate | 0,100 |
| Caprylyl/Capryl Glucoside | 0,150 |
| Ammonium Lactate | 0,100 |
| tert.-Butyl Alcohol | 0,1,00 |
| Aqua | ad 100,000 |

## Patentansprüche

1. Kosmetische oder dermatologische topische Zusammensetzung mit einem Gehalt an Benzoesäure und/oder ihren Salzen und Salicylsäure und/oder ihren Salzen, **dadurch gekennzeichnet, dass**
a) die Gesamtmenge an Benzoesäure und Benzoaten in der Zusammensetzung bezogen auf das Gewicht der Zusammensetzung 0,2 bis 0,7 Gew.-% beträgt und
b) die Gesamtmenge an Salicylsäure und Salicylaten in der Zusammensetzung bezogen auf das Gewicht der Zusammensetzung 0,2 bis 0,7 Gew.-% beträgt und
c) das Gewichtsverhältnis der Gesamtmenge an Benzoesäure und Benzoaten zur Gesamtmenge an Salicylsäure und Salicylaten im Bereich von 3 : 2 bis 2 : 3 liegt,
wobei sie bezogen auf ihr Gewicht 0,3 bis 0,65 Gew.-% Natriumbenzoat enthält und
wobei sie bezogen auf ihr Gewicht 0,3 bis 0,65 Gew.-% Natriumsalicylat enthält.

2. Zusammensetzung gemäß Anspruch 1, **dadurch gekennzeichnet, dass** sie bezogen auf ihr Gewicht 0,35 bis 0,6 Gew.-%, besonders bevorzugt 0,4 bis 0,6 Gew.-% und insbesondere 0,45 bis 0,5 Gew.-% Natriumbenzoat enthält.

3. Zusammensetzung nach einem der Ansprüche 1 oder 2, **dadurch gekennzeichnet, dass** sie bezogen auf ihr Gewicht 0,35 bis 0,6 Gew.-%, besonders bevorzugt 0,4 bis 0,6 Gew.-% und insbesondere 0,45 bis 0,5 Gew.-% Natriumsalicylat enthält.

4. Zusammensetzung nach einem der Ansprüche 1 bis 3, **dadurch gekennzeichnet, dass** das Gewichtsverhältnis der Gesamtmenge an Benzoesäure und Benzoaten zur Gesamtmenge an Salicylsäure und Salicylaten im Bereich von 5 : 4 bis 4 : 5 und insbesondere von 1,1 : 1 bis 1: 1,1 liegt.

5. Zusammensetzung gemäß einem der vorhergehenden Ansprüche, enthaltend bezogen auf ihr Gewicht
a) 20 bis 95 Gew.-% Wasser und
b) Dehydroxanthan Gum und
c) mindestens ein vernetztes Copolymer aus Vinylpyrrolidon und 2-Methyl-2[(1-oxo-2-propenyl)amino]-1-propansulfonsäure, das teilweise oder vollständig neutralisiert ist,
d) mindestens einen polymeren Verdicker in Form eines inversen, auto-inversiblen Polymerlatex, enthaltend eine Ölphase, eine Wasserphase, mindestens einen Öl-in-Wasser-Emulgator und mindestens einen verzweigten oder vernetzten Polyelektrolyten, wobei der Polyelektrolyt ausgewählt ist aus einem Copolymer, das mindestens ein Monomer mit einer starken Säurefunktion sowie ein Monomer mit einer schwachen Säurefunktion enthält.

6. Zusammensetzung gemäß Anspruch 5, **dadurch gekennzeichnet, dass** sie bezogen auf ihr Gewicht 30 bis 94 Gew.-%, vorzugsweise 35 bis 92,5 Gew.-%, besonders bevorzugt 40 bis 90 Gew.-%, weiter bevorzugt 50 bis 87,5 Gew.-% und insbesondere 60 bis 85 Gew.-% Wasser enthält.

7. Zusammensetzung gemäß einem der Ansprüche 5 oder 6, **dadurch gekennzeichnet, dass** sie bezogen auf ihr Gewicht 0,05 bis 5 Gew.-%, vorzugsweise 0,1 bis 4 Gew.-%, besonders bevorzugt 0,25 bis 3 Gew.-%, weiter bevorzugt 0,5 bis 2,5 Gew.-% und insbesondere 1 bis 2 Gew.-% Dehydroxanthan Gum b) enthält.

8. Zusammensetzung gemäß einem der Ansprüche 5 bis 7, **dadurch gekennzeichnet, dass** sie bezogen auf ihr Gewicht 0,05 bis 3 Gew.-%, vorzugsweise 0,1 bis 2 Gew.-%, besonders bevorzugt 0,2 bis 1,5 Gew.-%, weiter bevorzugt 0,25 bis 1,25 Gew.-% und insbesondere 0,5 bis 1 Gew.-% mindestens eines vernetzten Copolymers c) aus Vinylpyrrolidon und 2-Methyl-2[(1-oxo-2-propenyl)amino]-1-propansulfonsäure, das teilweise oder vollständig neutralisiert ist, enthält.

9. Zusammensetzung gemäß einem der Ansprüche 5 bis 8, **dadurch gekennzeichnet, dass** sie bezogen auf ihr Gewicht 0,1 bis 5 Gew.-%, vorzugsweise 0,25 bis 4 Gew.-%, besonders bevorzugt 0,5 bis 3 Gew.-%, weiter bevorzugt 0,75 bis 2,5 Gew.-% und insbesondere 1 bis 2 Gew.-% mindestens eines polymeren Verdickers d) in Form eines inversen, auto-inversiblen Polymerlatex enthält.

10. Zusammensetzung gemäß einem der Ansprüche 5 bis 9, **dadurch gekennzeichnet, dass** die schwache Säurefunktion des Polyelektrolytmonomeren im polymeren Verdicker d) eine Carboxylgruppe -COOH darstellt, die teilweise oder vollständig neutralisiert ist.

11. Zusammensetzung gemäß einem der Ansprüche 5 bis 10, **dadurch gekennzeichnet, dass** die Monomere des Polyelektrolyten im polymeren Verdicker d), die mit der schwach sauren -COOH-Gruppe funktionalisiert sind, ausgewählt sind aus Acrylsäure, Methacrylsäure, Itaconsäure und Maleinsäure, die teilweise oder vollständig neutralisiert sind.

12. Zusammensetzung gemäß einem der Ansprüche 5 bis 11, **dadurch gekennzeichnet, dass** das Polyelektrolytmonomer mit starker Säurefunktion im polymeren Verdicker d) ausgewählt ist aus 2-Methyl-2[(1-oxo-2-propenyl)amino]-1-propansulfonsäure, die teilweise oder vollständig neutralisiert ist.

13. Zusammensetzung gemäß einem der vorhergehenden Ansprüche, **dadurch gekennzeichnet, dass** sie bezogen auf ihr Gewicht 0,5 bis 5 Gew.-%, vorzugsweise 1,0 bis 4 Gew.-%, besonders bevorzugt 1,5 bis 3 Gew.-% und insbesondere 1,75 bis 2,5 Gew.-% Trimethylammoniumacetat (H₃C)₃N⁺CH₂COO⁻ enthält.

14. Zusammensetzung gemäß einem der vorhergehenden Ansprüche, **dadurch gekennzeichnet, dass** sie bezogen auf ihr Gewicht 0,25 bis 5 Gew.-%, vorzugsweise 0,5 bis 4 Gew.-%, besonders bevorzugt 0,75 bis 3 Gew.-% und insbesondere 1 bis 2 Gew.-% N,N'-Bis(2-hydroxyethyl)harnstoff enthält.

## Claims

1. A cosmetic or dermatological topical composition having a content of benzoic acid and/or the salts thereof and of salicylic acid and/or the salts thereof, **characterized in that**
a) the total amount of benzoic acid and benzoates in the composition, based on the weight of the composition, is from 0.2 to 0.7 wt %, and
b) the total amount of salicylic acid and salicylates in the composition, based on the weight of the composition, is from 0.2 to 0.7 wt.%, and
c) the weight ratio of the total amount of benzoic acid and benzoates to the total amount of salicylic acid and salicylates is in the range of from 3:2 to 2:3,
said composition containing based on the weight thereof, from 0.3 to 0.65 wt.% sodium benzoate, and
said composition containing, based on the weight thereof, from 0.3 to 0.65 wt.% sodium salicylate.

2. The composition according to claim 1, **characterized in that** it contains, based on the weight thereof, from 0.35 to 0.6 wt.%, particularly preferably from 0.4 to 0.6 wt.% and in particular from 0.45 to 0.5 wt.%, sodium benzoate.

3. The composition according to one of claims 1 or 2, **characterized in that** it contains, based on the weight thereof, from 0.35 to 0.6 wt.%, particularly preferably from 0.4 to 0.6 wt.% and in particular from 0.45 to 0.5 wt.%, sodium salicylate.

4. The composition according to one of claims. 1 to 3, **characterized in that** the weight ratio of the total amount of benzoic acid and benzoates to the total amount of salicylic acid and salicylates is in the range of from 5:4 to 4:5 and in particular from 1,1:1 to 1:1.1.

5. The composition according to one of the preceding claims, containing, based on the weight thereof,
a) from 20 to 95 wt.% water, and
b) dehydroxanthan gum, and
c) at least one cross-linked copolymer of vinylpyrrolidone and 2-methyl-2[(1-oxo-2-prapenyl)amino]-1-propane sulfonic acid which is partially or completely neutralized,
d) at least one polymeric thickener in the form of an inverse, self-invertible polymer latex, containing an oil phase a water phase, at least one oil-in-water emulsifier and at least one branched or cross-linked polyelectrolyte, wherein the polyelectrolyte is selected from a copolymer which contains at least one monomer having a strong acid function and one monomer having a weak acid function.

6. The composition according to claim 5, **characterized in that** it contains, based on the weight thereof, from 30 to 94 wt.%, preferably from 35 to 92.5 wit.%, particularly preferably from 40 to 90 wt.%, more preferably from 50 to 87.5 wt.% and in particular from 60 to 85 wt.%, water,

7. The composition according to one of claims 5 or 6, **characterized in that** it contains, based on the weight thereof, from 0.05 to 5 wt.%, preferably from 0.1 to 4 wt.%, particularly preferably from 0.25 to 3 wt.%, more preferably from 0.5 to 2.5 wt.% and in particular from 1 to 2 wt %, dehydroxanthan gum b).

8. The composition according to one of claims 5 to 7, **characterized in that** it contains, based on the weight thereof, from 0.05 to 3 wt.%, preferably from 0.1 to 2 wt.%, particularly preferably from 0.2 to 1.5 wt.%, more preferably from 0.25 to 1.26 wt.% and in particular from 0.5 to 1 wt.%, of at least one cross-linked copolymer c) of vinylpyrrolidone and 2-methyl-2[(1-oxo-2-propenyl)amino]-1-propane sulfonic acid which is partially or completely neutralized.

9. The composition according to one of claims 5 to 8, **characterized in that** it contains, based on the weight thereof, from 0.1 to 5 wt.%, preferably from 0.26 to 4 wt.%, particularly preferably from 0.5 to 3 wt.%, more preferably from 0.75 to 2.5 wt.% and in particular from 1 to 2 wt.%, of at least one polymeric thickener d) in the form of an inverse, self-invertible polymer latex.

10. The composition according to one of claims 5 to 9, **characterized in that** the weak acid function of the polyelectrolyte monomer in the polymeric thickener d) is a carboxyl group -COOH which is partially or completely neutralized.

11. The composition according to one of claims 5 to 10, **characterized in that** the monomers of the polyelectrolyte in the polymeric thickener d), which are functionalized with the weak acid -COOH-group, are selected from acrylic acid, methacrylic acid, itaconic acid and maleic acid which are partially or completely neutralized.

12. The composition according to one of claims 5 to 11, **characterized in that** the polyelectrolyte monomer having a strong acid function in the polymeric thickener d) is selected from 2-methyl-2[(1-oxo-2-propenyl)arnino]-1-propane sulfonic acid which is partially or completely neutralized.

13. The composition according to one of the preceding claims, **characterized in that** it contains, based on the weight thereof, from 0.5 to 5 wit.%, preferably from 1.0 to 4 wt.%, particularly preferably from 1.5 to 3 wt.% and in particular from 1.75 to 2.5 wt.%, trimethylammonium acetate (H₃C)₃N⁺CH₂COO⁻.

14. The composition according to one of the preceding claims, **characterized in that** it contains, based on the weight thereof, from 0.25 to 5 wt.%, preferably from 0.5 to 4 wt.%, particularly preferably from 0.75 to 3 wit.% and in particular from 1 to 2 wt.%, N,N'-bis(2-hydroxyethyl)urea.

## Revendications

1. Composition cosmétique ou dermatologique avec une teneur en acide benzoïque ef/ou en ses sels et en acide salicylique et/ou en ses sels, **caractérisée en ce que** :
a) la quantité totale en acide benzoïque ou en benzoates dans la composition, rapportée au poids de la composition, est de 0,2 à 0,7 % en poids, et
b) la quantité totale en acide salicylique et en salicylates dans la composition rapportée au poids de la composition, est de 0,2 à 0,7 % en poids, et
c) le rapport pondéral de la quantité totale en acide benzoïque ou en benzoates sur la quantité totale en acide salicylique et en salicylates est entre 3:2 et 2:3
la composition contenant 0,3 à 0,65 % en poids de benzoate de sodium, rapporté à son poids, et
la composition contenant 0,3 à 0,65 % en poids de salicylate de sodium, rapporté à son poids.

2. Composition selon la revendication 1, **caractérisée en ce qu'**elle contient, rapporté à son poids, 0,35 à 0,6 % en poids, particulièrement préférentiellement 0,4 à 0,6 % en poids et en particulier 0,45 à 0,5 % en poids de benzoate de sodium.

3. Composition selon une des revendications 1 ou 2, **caractérisée en ce qu'**elle contient, rapporté à son poids, 0,35 à 0,6 % en poids, particulièrement préférentiellement 0,4 à 0,6 % en poids et en particulier 0.45 à 0,5 % en poids de salicylate de sodium.

4. Composition selon une des revendications 1 à 3, **caractérisée en ce que** le rapport pondéral de la quantité totale d'acide benzoïque et de benzoates à la quantité totale d'acide salicylique et de salicylates se situe entre 5:4 et 4:5, et en particulier entre 1,1:1 et 1:1,1.

5. Composition selon une des revendications précédentes, contenant, rapporté à son poids :
a) 20 à 95 % en poids d'eau, et
b) de la gomme de xanthane déshydratée
c) au moins un copolymère réticulé de vinylpyrrolidone et d'acide 2-mathyl-2((1-oxo-2-propényl)amino]-1-propanesulfonique, partiellement ou totalement neutralisé,
d) au moins un épaississant polymère sous forme d'un latex polymère inverse, auto-inversible, contenant une phase huileuse, une phase aqueuse, au moins un émulsifiant huile dans l'eau et au moins un polyélectrolyte ramifié ou réticulé, le polyélectrolyte étant choisi parmi un copolymère contenant au moins un monomère avec une fonction acide forte ainsi qu'un monomère avec une fonction acide faible.

6. Composition selon la revendication 5, **caractérisée en ce qu'**elle contient, rapporté à son poids, 30 à 94 % en poids, de préférence 35 à 92,5 % en poids, particulièrement préférentiellement 40 à 90 % en poids, plus préférentiellement 50 à 87,5 % en poids et en particulier 60 à 85 % en poids d'eau.

7. Composition selon une des revendications 5 ou 6, **caractérisée en ce qu'**elle contient, rapporté à son poids, 0,05 à 5 % en poids, de préférence 0,1 à 4 % en poids, particulièrement préférentiellement 0,25 à 3 % en poids, plus préférentiellement 0,5 à 2,5 % en poids et en particulier 1 à 2 % en poids de gomme de xanthane déshydratée b).

8. Composition selon une des revendications 5 à 7, **caractérisée en ce qu'**elle contient, rapporté à son poids, 0,05 à 3% en poids, de préférence 0,1 à 2 % en poids, particulièrement préférentiellement 0,2 à 1,5 % en poids, plus préférentiellement 0,25 à 1,25 % en poids et en particulier 0,5 à 1 % en poids d'au moins un copolymère réticulé c) de vinylpyrrolidone et d'acide 2-méthyl-2((1-oxo-2-propényl)aminol]-1-propenesulfonique, partiellement ou totalement neutralisé.

9. Composition selon une des revendications 5 à 8, **caractérisée en ce qu'**elle contient, rapporté à son poids, 0,1 à 5 % en poids, de préférence 0,25 à 4% en poids, particulièrement préférentiellement 0,5 à 3 % en poids, plus préférentiellement 0,75 à 2,5 % en poids et en particulier 1 à 2% en poids d'au moins un épaississant polymère d) sous forme d'un latex polymère inverse, auto-inversible.

10. Composition selon une des revendications 5 à 9, **caractérisée en ce que** la fonction acide faible du monomère polyélectrolyte représente dans l'épaississeur polymère d) un groupe carboxyle -COOH partiellement ou totalement neutralisé.

11. Composition selon une des revendications 5 à 10, **caractérisée en ce que** les monomères du polyélectrolyte dans l'épaississant polymère d), qui sont fonctionnalisés avec le groupe acide faible -COOH, sont choisis parmi l'acide acrylique, l'acide méthacrylique, l'acide itaconique, et l'acide maléique, partiellement ou totalement neutralisé.

12. Composition selon une des revendications 5 à 11, **caractérisée en ce que** le monomère polyélectrolyle avec une fonction acide forte dans l'épaississant polymère d), est choisi parmi l'acide 2-méthyl-2[(1-oxo-2-propényl)amino]-1-propanesulfonique partiellement ou totalement neutralisé.

13. Composition selon une des revendications précédentes, **caractérisée en ce qu'**elle contient, rapporté à son poids, 0,5 à 5 % en poids, de préférence 1,0 à 4 % en poids, particulièrement préférentiellement 1,5 à 3% en poids et en particulier 1,75 à 2,5 % en poids d'acétate de triméthylammonium (H₃C)₅N⁺CH₂COO-.

14. Composition selon une des revendications précédentes, **caractérisée en ce qu'**elle contient, rapporté à son poids, 0,25 à 5 % en poids, de préférence 0,5 à 4 % en poids, particulièrement préférentiellement 0,75 à 3 % en poids et en particulier 1 à 2 % en poids de N,N'-bis-(2-hydroxyéthyl)urée.
